# EUROPEAN PATENT SPECIFICATION

(11) **EP 0 862 629 B1**
(45) Date of publication and mention of the grant of the patent: **01.08.2001**
(21) Application number: 96940865.7
(22) Date of filing: 22.11.1996
(51) Int. Cl.: C12N 15/12, C07K 14/705, C07K 16/30, A61K 48/00, C12Q 1/68, G01N 33/50, C12N 15/11

(54) **EPITHELIAL MEMBRANE PROTEIN-1**
EPITHELIALES MEMBRANPROTEIN-1
PROTEINE-1 DE LA MEMBRANE EPITHELIALE

(30) Priority: 23.11.1995 US 563839
(43) Date of publication of application: 09.09.1998
(73) Proprietor: Amgen Inc., Thousand Oaks, California 91320-1799 (US); Eidgenössische Technische Hochschule (ETH) Zürich, 8092 Zürich (CH)
(72) Inventor: TAYLOR, Verdon, CH-8046 Zurich (CH); WELCHER, Andrew, A., Thousand Oaks, CA 91320 (US); SUTER, Ueli, CH-5436 Wuerenlos (CH)
(74) Representative: Vossius, Volker, Dr.
(86) International application number: US9618852
(87) International publication number: WO9719171

(56) References cited:
- J BIOL CHEM, DEC 1 1995, 270 (48) P28824-33, UNITED STATES, XP000645327 TAYLOR V ET AL: "Epithelial membrane protein-1, peripheral myelin protein 22, and lens membrane protein 20 define a novel gene family."
- GENOMICS, SEP 15 1996, 36 (3) P379-87, UNITED STATES, XP000645625 LOBSIGER CS ET AL: "Identification and characterization of a cDNA and the structural gene encoding the mouse epithelial membrane protein-1."
- JOURNAL OF IMMUNOLOGY, vol. 157, no. 1, 1 July 1996, pages 72-80, XP000616417 RUEGG C L ET AL: "B4B, A NOVEL GROWTH-ARREST GENE, IS EXPRESSED BY A SUBSET OF PROGENITOR/PRE-B LYMPHOCYTES NEGATIVE FOR CYTOPLASMIC MU-CHAIN"
- JOURNAL OF BIOLOGICAL CHEMISTRY, vol. 270, no. 48, 1 December 1995, pages 28910-28916, XP000616413 MARVIN K W ET AL: "IDENTIFICATION AND CHARACTERIZATION OF A NOVEL SQUAMOUS CELL-ASSOCIATED GENE RELATED TO PMP22"
- PROCEEDINGS OF THE NATIONAL ACADEMY OF SCIENCES OF USA, vol. 88, 1991, WASHINGTON US, pages 7195-7199, XP002027824 WELCHER AA ET AL.: "A myelin protein is encoded by the homologue of a growth arrest-specific gene" cited in the application
- BEN-PORATH I. ET AL: "Mus musculus tumor associated membrane protein (TMP) mRNA, complete CDs.", EMBL Database entry MMMP. Accession number U25633; 24-08-1995

## Description

The present invention relates to transmembrane proteins which act in signal transduction to modulate the growth and/or differentiation of target cells. More particularly, the invention relates to epithelial membrane protein-1 (EMP-1), recombinant production of EMP-1, and methods and reagents for modulating the activity and expression of EMP-1.

### Background of the Invention

An understanding of the mechanisms involved in cellular growth and differentiation promises to be useful in elucidating the causes of and providing treatments for human disease. One area of research has focused on identification of cell surface receptors which act in signal transduction. Cell surface receptors are transmembrane proteins which transfer a stimulus from the outside environment of the cell to intracellular molecules which in turn impart a change in cell physiology. This change in physiology is typically one involving the growth or differentiation state of the cell. A number of receptor families consisting of structurally and functionally related members have been identified (see review by Van der Geer et al. Ann. Rev. Cell Biol. 10, 251-337 (1994).

Generally cell surface receptors transduce a signal to the cell interior upon interaction with a ligand. The ligand, usually a polypeptide, typically induces a conformational change in the receptor which triggers the signalling process. This process is referred to as receptor activation and may involve events such as receptor oligomerization (either with the same or with different receptors) and receptor tyrosine phosphorylation. While polypeptide ligands studied so far will preferentially interact with a single receptor a given ligand may also bind to and activate one or more other members within a receptor family.

Techniques for rapidly isolating and sequencing DNA have greatly accelerated the search for receptors and their cognate ligands. In particular, random sequencing of cDNA libraries to generate expressed sequence tags (ESTs) along with computational methods which compare newly obtained ESTs with nucleic acid sequences in sequence databases now permit the rapid identification of sequences having high degrees of homology. It is now possible to rapidly assign a new sequence (full or partial) to a family of related sequences and, in doing so, to deduce one or more likely functions of the encoded proteins. In addition, knowledge of highly conserved stretches of nucleic acid or amino acid sequence in families of related sequences allows one to design oligonucleotide probes for screening cDNA libraries for related molecules.

The identification of related members of a single family of receptors or ligands provides valuable insight into the structure and function of that family. Related members of a family lead to an understanding of the role of receptor family members, their cognate ligands, and accessory proteins in cellular physiology. This information aids in the development of therapies based upon the stimulation or blocking of receptor and/or ligand functions.

It is an object of the invention to identify sequences which encode receptors or their cognate ligands. It is a further object to understand structure/function relationships of new receptor or ligand sequences to existing sequences. Isolated nucleic acid sequences have been found encoding an epithelial membrane protein-1 (EMP-1) that is structurally related to peripheral myelin protein-22 (PMP-22) and along with PMP-22 defines a new family of receptors.

PMP22, or peripheral myelin protein 22, is a transmembrane protein that has been previously identified as an expression product primarily in Schwann cells (Welcher et al. Proc. Natl. Acad. Sci. USA 88, 7195-7199 (1991); Spreyer et al. EMBJ. 10, 3661-3668 (1991)). Point mutations or gene arrangements in PMP22 or abnormal expression of the corresponding gene lead to motor and sensory neuropathies of the peripheral nervous system (PNS) (Suter et al., TINS 16, 50-56 (1993)). Human diseases associated with alterations in the PMP22 gene include a variety of neuropathies such as Charcot-Marie-Tooth disease type 1A and Dejerine-Sottas syndrome. (Matsunami et al. Nature Genet. 1, 176-179 (1992); Patel et al. Nature Genet. 1, 159-165 (1992); Roa et al. Nature Genet. 5, 269-272 (1993)). In addition, PMP22 was observed to be upregulated under growth arrest conditions in cell culture, suggesting a role for PMP22 in cell proliferation. However, no known PMP22 mutants show any phenotype other than that observed in the PNS, suggesting that related molecules may compensate for the lack of functional PMP22 in non-neural tissues. Further, there has been no significant homology observed between the amino acid sequence of PMP22 and any other known protein. A description of PMP22 and its involvement in PNS disorders has been described in PCT Application No. 92/21694 (= EP No. 92913617.4).

The nucleotide sequence of the EMBL Database entry MMMP, Accession number U25633, created on August 24, 1995 describes a mRNA sequence of tumor-associated membrane protein (TMP) from Mus musculus.

The identification of PMP22-related molecules provides potential therapies based upon regulation of the growth and/or differentation of tissues which bear PMP22 related molecules on their surface and which respond to external stimuli recognized by PMP22-related molecules. Identification of PMP22-related molecules allows one to design compounds for the control of cell growth and/or differentiation.

### Summary of the Invention

The present invention provides for epithelial membrane protein-1 (EMP-1) which has significant sequence homology to PMP-22 and along with PMP-22 defines a new family of receptors. EMP-1 is involved in the control of growth and/or differentiation of the various tissues in which it is expressed. In particular, EMP-1 may function in the nervous system, lung and in the epithelial cells lining the gastrointestinal tract.

Isolated nucleic acid molecules encoding EMP-1 are provided. EMP-1 is preferably mammalian in origin and may be mouse, rat or human. The nucleic acids may also be variants which are naturally occurring or constructed by *in vitro* mutagenesis of the EMP-1 nucleic acid sequence. DNA molecules comprising an expression system capable of expressing EMP-1 and host cells modified with said DNA molecules are also encompassed. Preferably, the modified host cells will display EMP-1 at the cell surface. The nucleic acids may also be used in gene therapy or anti-sense therapy for treatment of disorders related to EMP-1 as well as disorders related to EMP-1 expressing cells.

A method for producing host cells displaying EMP-1 is also provided by the invention. Said host cells may be used to screen for agonists or antagonists of EMP-1 and for proteins which interact with EMP-1.

### Description of the Figures

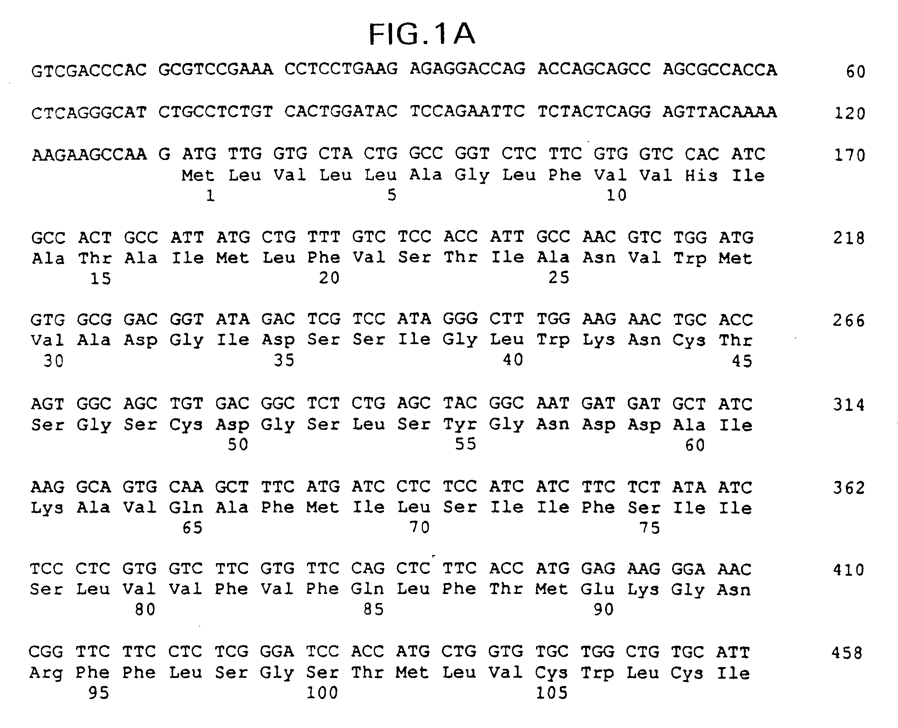

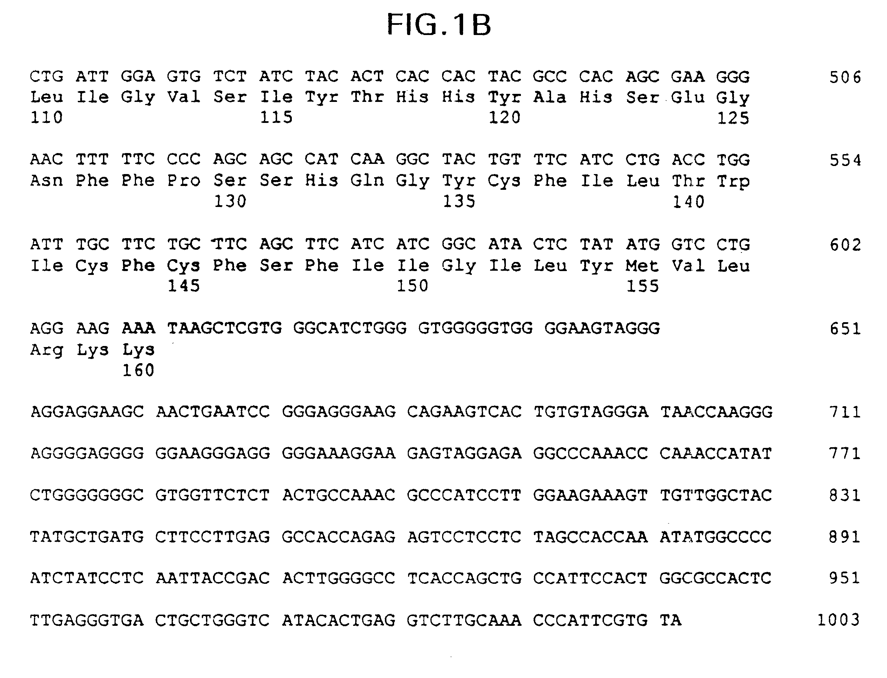

Figure 1. cDNA and predicted amino acid sequence of rat EMP-1. The rat EMP-1 cDNA clone contains an open reading frame of 480 base pairs starting with an ATG codon at nucleotide 132 and terminating with a TAA stop codon at position 612. A potential cleavable signal peptide spans amino acid residues 1 to 16. A single motif for putative N-linked glycosylation is present between the first and second hydrophobic domains at asparagine 43.

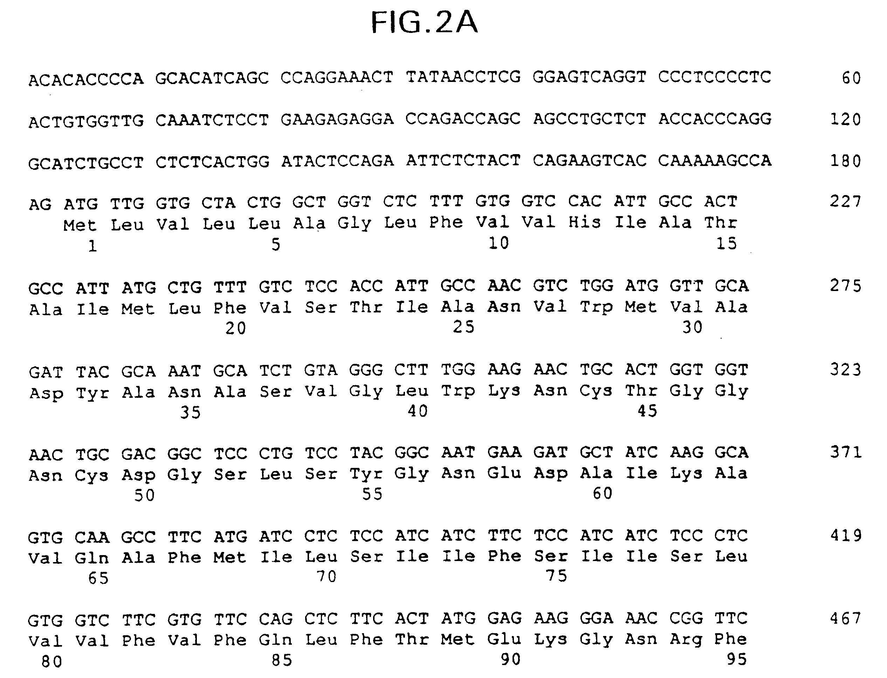

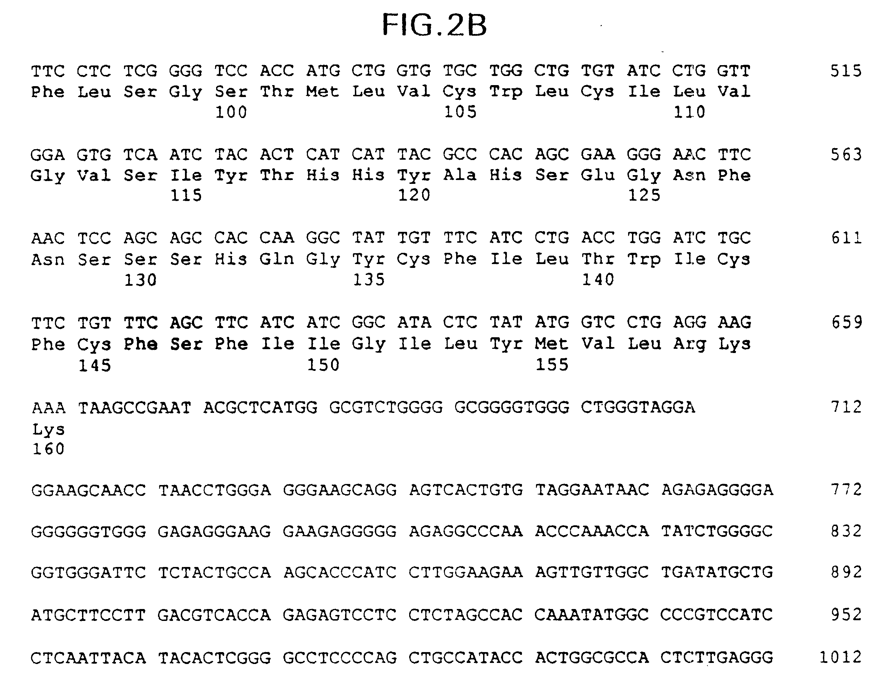

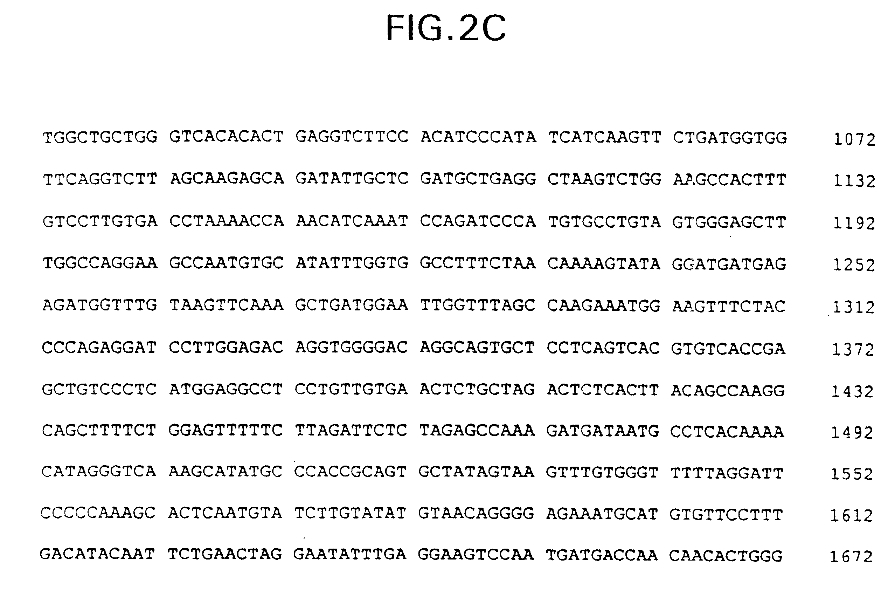

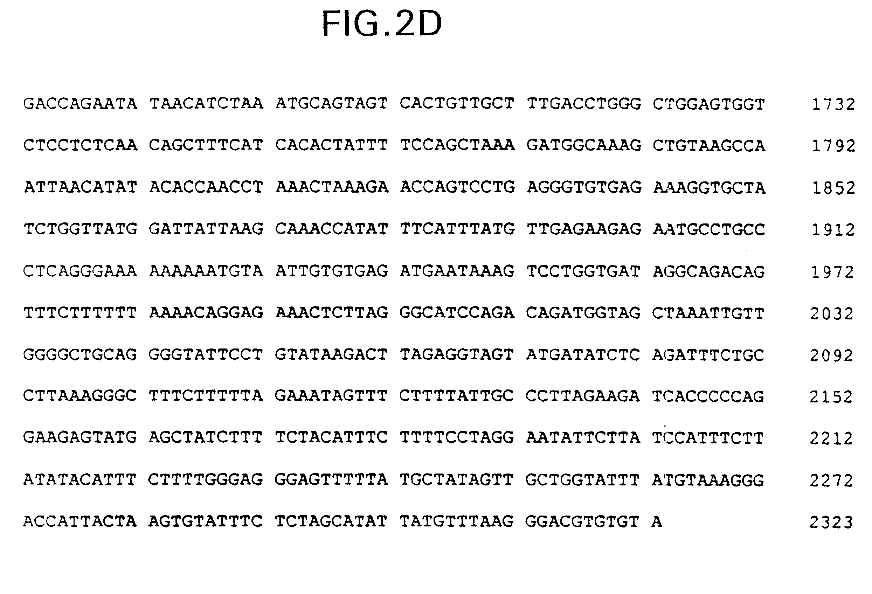

Figure 2. cDNA and predicted amino acid sequence of mouse EMP-1. The mouse cDNA clone contains as open reading frame of 480 base pairs starting with an ATG codon at nucleotide 183 and terminating with a TAA codon at nucleotide 663. A potential cleavable signal peptide spans amino acid residues 1 to 16. The potential N-linked glycosylation site is conserved from the rat sequence.

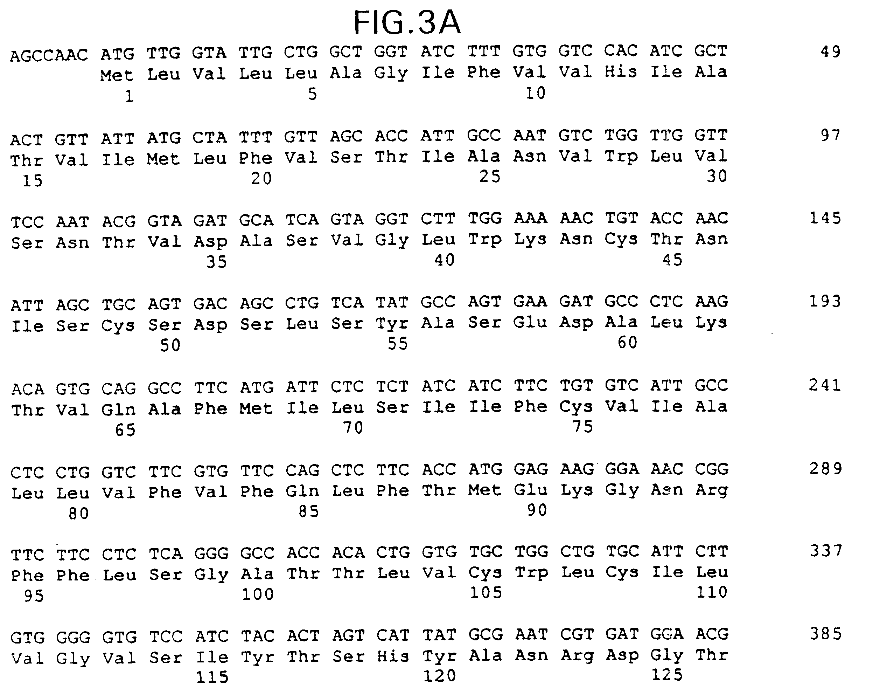

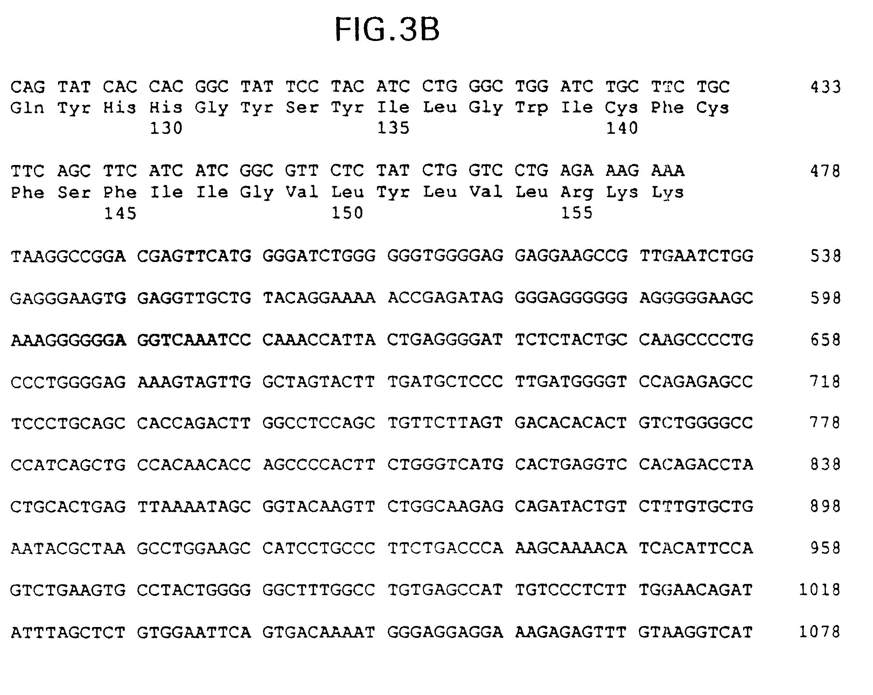

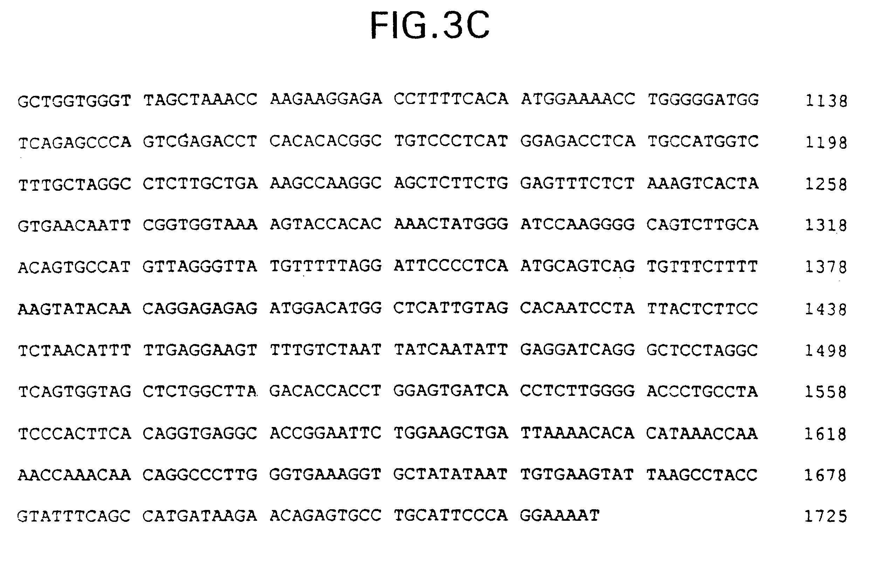

Figure 3. cDNA and predicted amino acid sequence of human EMP-1. The human cDNA clone contains an open reading frame of 471 base pairs starting with an ATG codon at nucleotide 8 and terminating with a TAA stop codon at position 479. The potential signal peptide spans residues 1 to 16. The potential N-liked glycosylation site is conserved from the rat and mouse sequences.

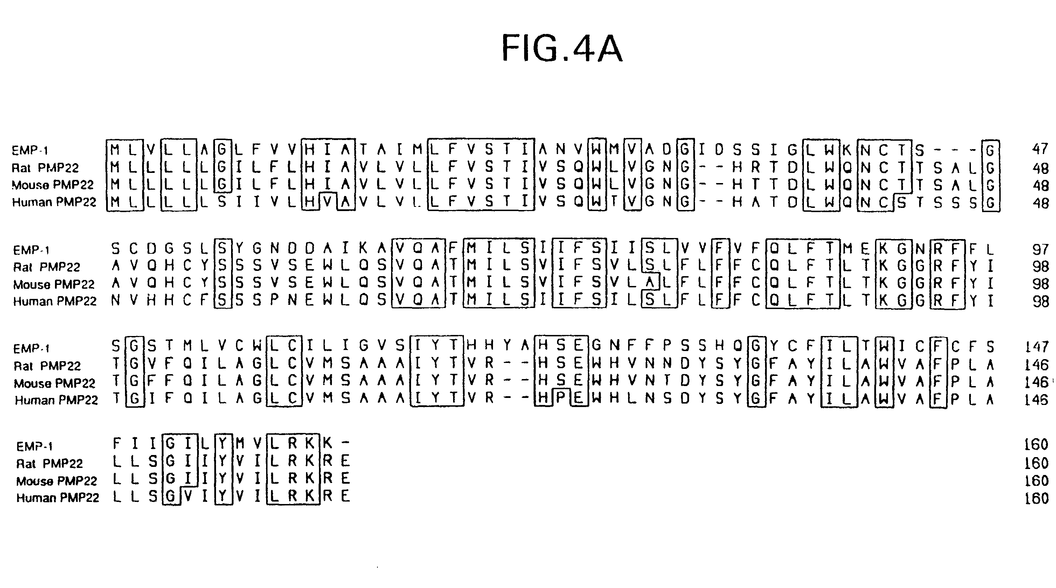

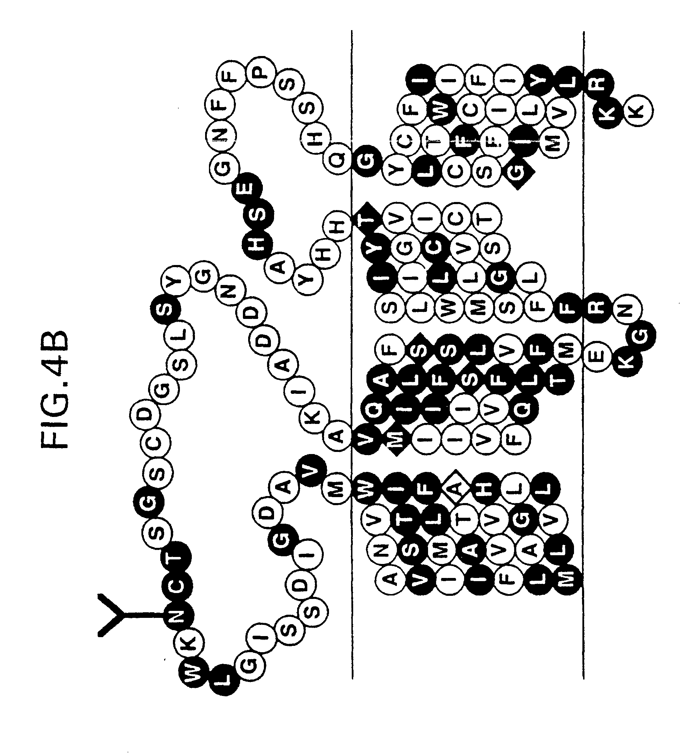

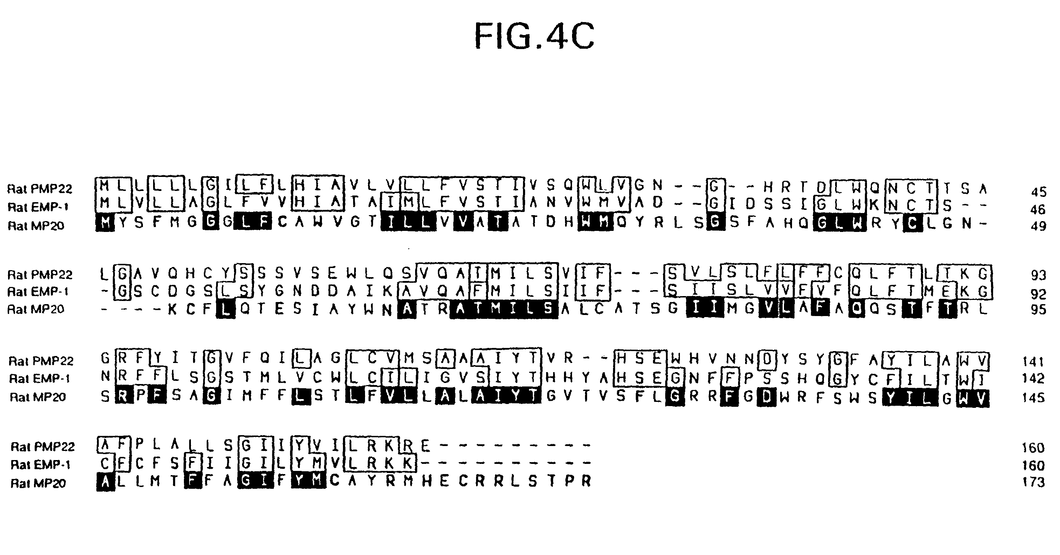

Figure 4. Predicted EMP-1 structure and amino acid sequence comparison of PMP22/EMP family members. **A**. Comparison of predicted amino acid sequences of rat EMP-1 with rat, mouse and human PMP22. **B.** Hypothetical topology of EMP-1 in a lipid bilayer based on computer-assisted hydrophobicity plots and secondary structure predictions. Residues identical to rat PMP22 are filled. Most of the point mutations in PMP22 known to result in hereditary peripheral neuropathies (diamonds) are conserved at the corresponding positions in EMP-1. The Y-shaped symbol indicates a potential N-linked carbohydrate chain. **C.** Amino acid comparison of the three known PMP22/EMP/MP20 family members. The residue conserved between MP20 and PMP22 or EMP-1 as shown in reversed type.

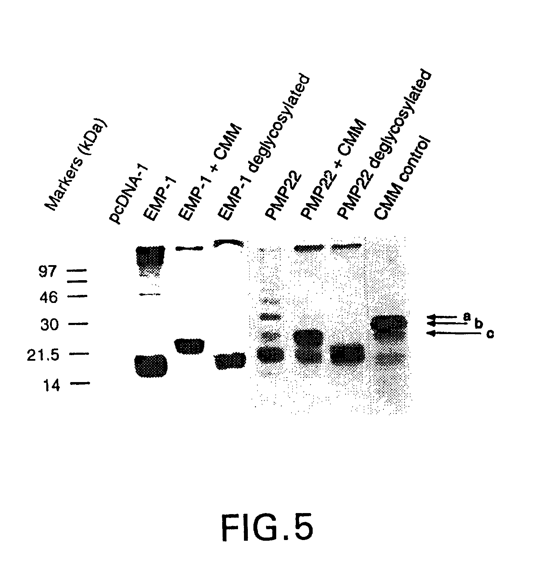

Figure 5. *In vitro* transcription and translation of EMP-1 and PMP22 cDNAs. The [³⁵S]methionine metabolically labelled proteins were separated by reducing 15% SDS PAGE. pcDNA-1 was used as a control and no specific proteins can be detected. The EMP-1 cDNA generated an 18 kDa protein which clusters (EMP-1). Transcription and translation of the EMP-1 cDNA in the presence of CMM results in a reduced rate of migration of the protein (EMP-1 + CMM). This reduced migration is reversed by deglycosylation with N-Glycosidase F (EMP-1 deglycosylated). The PMP22 cDNA generates an 18 kDa protein whose apparent molecular weight increases by 4-6 kDa when the reaction is performed in the presence of CMM (PMP22 + CMM). Treatment with N-Glycosidase F reduced the molecular weight back to 18 kDa (PMP22 deglycosylated). Neither EMP-1 nor PMP22 are substrates for signal peptidase *in vitro* as indicated by the identical migration rate of the unglycosylated (EMP-1, PMP22) and deglycosylated translation products (EMP-1 + CMM, PMP22 + CMM). Prolactin **(a)** is a substrate for signal peptidase, approximately 50% of the protein is processed when translated in the presence of CMM **(c)** (Promega Technical manual, Promega Biotech, Madison, WI). α-factor was used as a control for N-linked glycosylation and was completely modified in the reactions **(b)** (Promega Technical manual),.

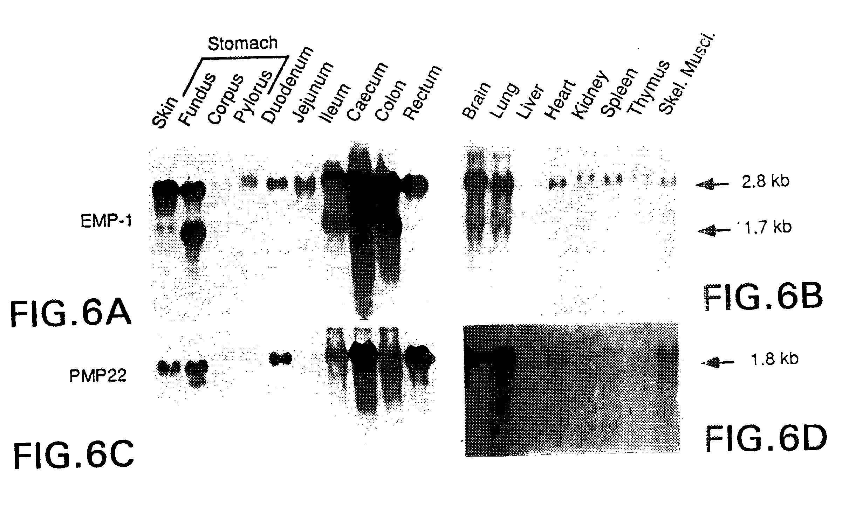

Figure 6. Tissue distribution of EMP-1 and PMP22 mRNAs in the rat. Northern blot analysis with a radiolabelled EMP-1 probe shows high expression of 2.8 kb transcripts in the caecum, colon, rectum, fundus and ileum **(a)**. Lower levels of expression are observed in the duodenum and jejunum of the small intestine and the corpus and pylorus of the stomach **(a)**. Additional transcripts of 1.7 kb are found in the fundus, ileum, caecum and colon **(a)**. In extraintestinal tissues, EMP-1 mRNA levels are high in the skin, whereas in the brain and lung, expression is comparable to the duodenum (**b**; panels **a** and **c** are 20 hour exposures, and **b** and **d** 48 hours exposures of the same blot). PMP22 mRNA is also highly expressed in the intestine **(c)**; its 1.8 kb transcript is most prominent in the rectum and caecum where expression is comparable to that of PMP22 in the lung (**c**, **d**). Ten µg of total RNA was loaded per lane and equal loading was verified by ethidium bromide staining.

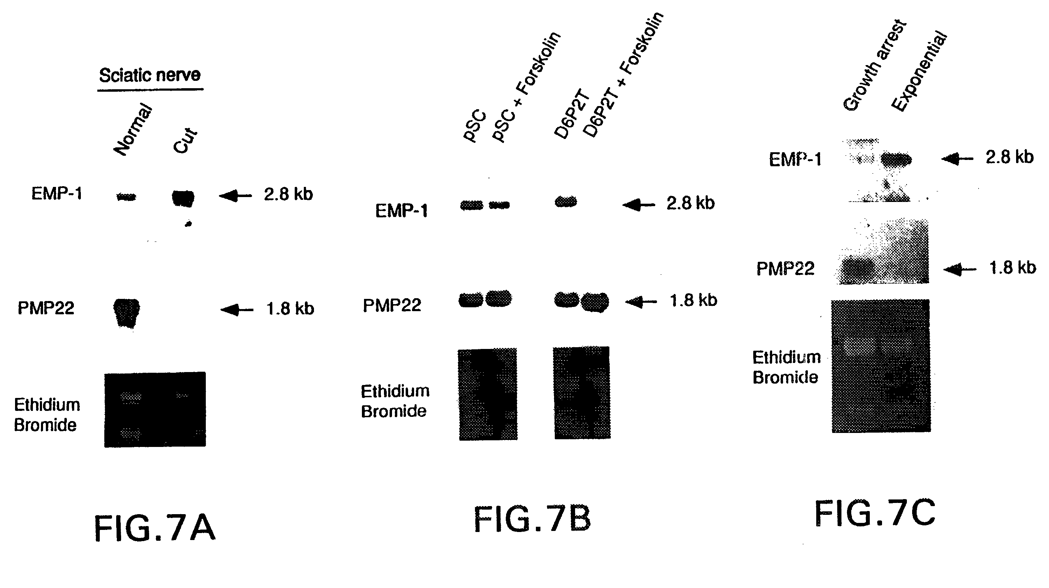

Figure 7. Regulation of EMP-1 and PMP22 mRNA expression by sciatic nerve injury and in cultured cells. **A.** Northern blot analysis of EMP-1 mRNA reveals an increased expression in the degenerating distal part of the injured sciatic nerve (4 days after nerve cut) compared to normal control nerve. PMP22 expression is considerably higher than that of EMP-1 in the normal nerve (1 hour exposure using the PMP22 probe compared to 36 hours for the EMP-1 probe). In contrast to EMP-1, PMP22 mRNA is dramatically reduced in the distal nerve after injury. **B.** Cultured, mitogen-expanded primary rat Schwann cells (pSC) and D6P2T Schwann cells display reduced EMP-1 expression following forskolin treatment. In contrast, PMP22 mRNA expression is increased under the same conditions. **C**. Serum starvation-induced growth arrest of NIH3T3 cells results in reduced EMP-1 mRNA expression and an increase in PMP22 expression relative to exponentially growing cells. Northern blot analyses were performed on the same blot (10 µg total RNA per sample) which was stripped between hybridizations.

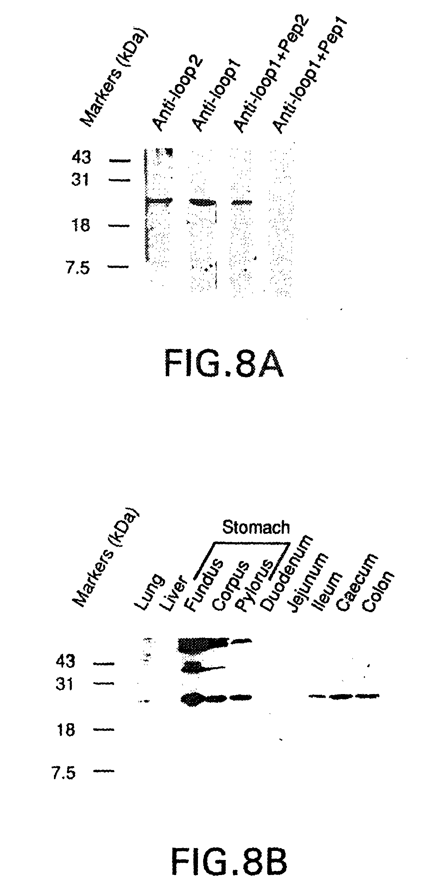

Figure 8. Expression of EMP-1 protein in the rat intestine. **A.** Two rabbit anti-EMP-1 peptide antisera raised against each of the putative extracellular loops 1 and 2 of EMP-1 recognize a 25 kDa protein in the corpus gastricum (50 µg protein lysate analysed by 12% SDS-PAGE and Western blotting). The immune reactivity of the anti-loopl antiserum was blocked by preincubation with 250 µg/ml of the immunogen but not by the loop2 peptide. **B.** Strong expression of EMP-1 protein is found in the stomach and large intestine, lower levels are present in the lung. Detection of a signal in the small intestine requires prolonged reaction time of the enzymatic detection system.

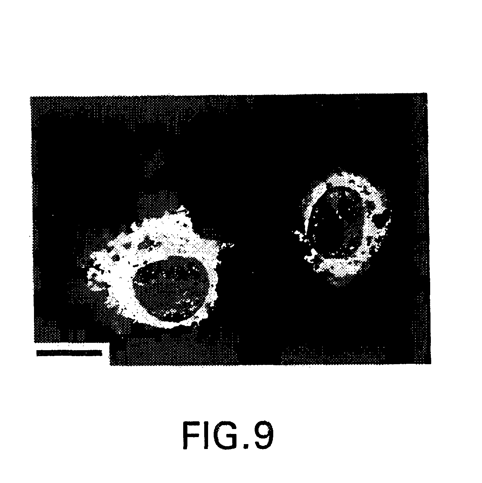

Figure 9. Detection of transiently expressed EMP-1 in COS cells. COS cells were transiently transfected with an EMP-1 expression construct and subsequently analysed by immunofluorescence using the anti-loop2 antiserum and a FITC-labelled goat anti-rabbit Ig. Identical results were obtained using the anti-loop1 antiserum. The scale bar represents 20 µm.

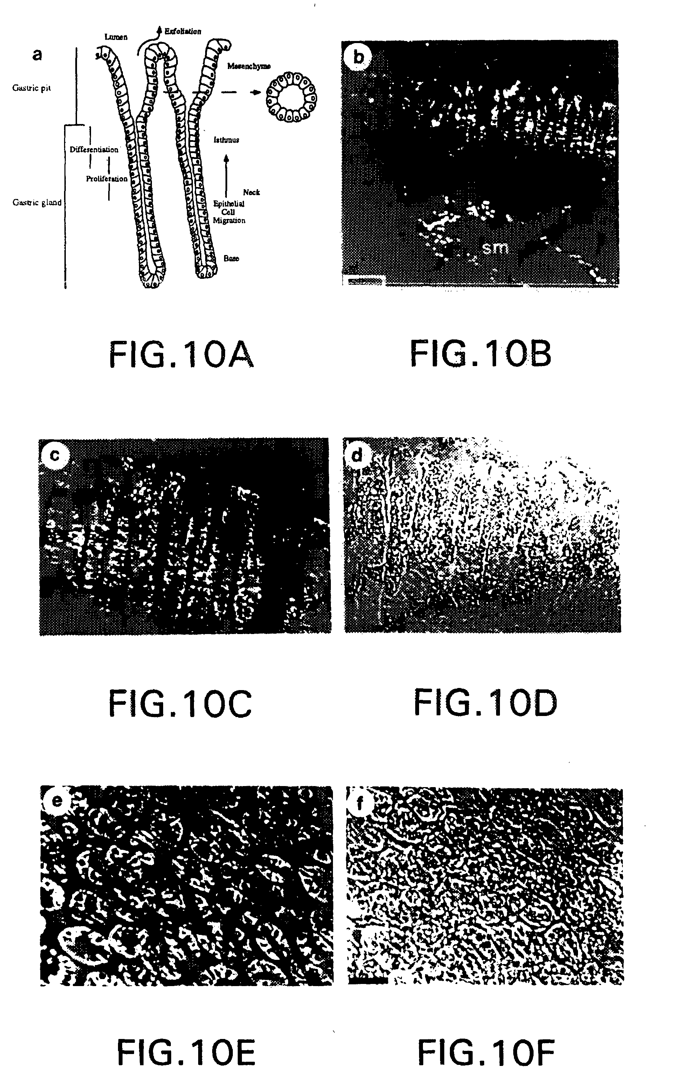

Figure 10. Immunofluorescent localization of EMP-1 protein expression in the corpus gastricum. **a)** Schematic view of the gastric mucosa showing the proliferative zone in the neck/isthmus region of the gastric gland and the migration of the differentiating epithelial cells towards the gastric pit. **b)** Low magnification view of the gastric mucosa labelled with the polyclonal anti-EMP-1 loop2 antiserum and detected with a Texas Red-labelled donkey anti-rabbit antibody. Intense immunoreactivity can be detected in the epithelial cells of the outer mucosa. No immunoreactive cells can be found towards the base of the gastric pit or in the sub-mucosal muscle layer (sm). The intense labelling of the isolated cells at the base of the gastric mucosa is not specific as it is also present in control sections incubated with preimmune serum (not shown). **c)** Higher magnification of the labelled epithelial cells in the pit region. The migrating, differentiating epithelial cells in the isthmus express high levels of EMP-1 protein. **d)** Transmitted light view of the region shown in panel c. **e)** Cross section through the gastric pit shows intense plasmamembrane-associated labelling of the epithelial cells but no labelling of the mesenchyme. No staining is seen in transverse sections across the base of the gastric gland (not shown). **f)** Transmitted light view of the section shown in panel e. The scale bars shown are 100 µm for **b** and 60 µm for panels c to **f.** sm- sub mucosal muscle layer.

### Detailed Description of the Invention

cDNA libraries can be sequenced at random to generate rapidly many partial sequences referred to as expressed sequence tags (ESTs). Two ESTs obtained from a mouse and rat cDNA library were observed to have significant homology with the corresponding PMP22 DNA sequences. Further analysis revealed the full length coding sequences for the protein, termed epithelial membrane protein-1 (EMP-1). These sequences were used as probes to isolate the corresponding human sequence by screening a human lung cDNA library.

Computer-assisted analysis predicted the following properties for rat, mouse and human EMP-1. The proteins are highly hydrophobic, and likely contain four transmembrane regions. The transmembrane domains in rat EMP-1 are predicted to span amino acid residues 1-28, 64-89, 95-117 and 134-157. Two predicted extracellular domains encompass the amino acid residues 29-63 and 113-133. These proteins are therefore integral membrane proteins and are likely to be either a receptor or a channel. The only sequence that showed homology to these proteins in the GenBank database is PMP22. EMP-1 and PMP22 show 40% amino acid identity and a very striking conservation of predicted structure. Comparison across EMP-1 species showed that the mouse and human protein had 78% identity. Based on these comparisons, PMP22, EMP-1 and the previously cloned lens membrane protein 20 (MP20) define a new family of proteins.

The high degree of identity at the amino acid level suggests that EMP-1 and PMP22 may serve similar functions. Close examination of the amino acid sequences of these proteins reveals that the hydrophobic regions, in particular the first two transmembrane domains, are highly conserved suggesting that they are of particular functional importance. This hypothesis is further supported by the finding that the hydrophobic domains are the most strongly conserved regions between PMP22 species homologues. Interestingly, the amino acid residues in PMP22 that are sites of mutation in hereditary peripheral neuropathies are located within putative transmembrane domains and the majority of these mutated amino acid residues are also conserved at the corresponding positions of EMP-1 and MP20.

A conserved feature within the putative extracellular domains of EMP-1 and PMP22 is the consensus sequence for an N-linked glycosylation. This glycosylation site in PMP22 carries a modified carbohydrate chain containing the L2/HNK-1 epitope, a structure which has been implicated in cell-cell recognition and adhesion processes (for recent review see Schachner et al., TINS 18, 183-191 (1995)). Although the presence and nature of carbohydrate moieties linked to EMP-1 remains to be determined, an N-linked glycosylation in the identical position of EMP-1 may be involved in cell recognition processes in the epithelium of the intestine.

### Expression of EMP-1 and PMP22

The tissue distribution of mRNA for rat EMP-1 and rat PMP22 is shown in Figure 6. EMP-1 mRNA is highly expressed in the adult digestive system including cecum, colon, ileum, jejunum, colonic crypts, duodenum, and stomach. Lower level expression is also seen in most other tissues including the skin, heart, brain, thymus, lung, and kidney. The expression in the digestive system is similar to that seen for PMP22. Although EMP-1 mRNA is expressed in peripheral nerve, it is expressed at much lower levels than PMP22. EMP-1 mRNA is expressed in a broader range of tissues than is PMP22. EMP-1 mRNA was detected in most human adult tissues examined, including heart, brain, lung, muscle, kidney, colon, and small intestine.

Regulation of EMP-1 and PMP22 expression was studied in several different systems. In cut sciatic nerve, where Schwann cells are continually dividing, EMP-1 mRNA is increased, while PMP22 is decreased. In Schwann cells (both primary and cell lines), EMP-1 mRNA is downregulated following forskolin treatment, while PMP22 is increased. In fibroblasts, EMP-1 mRNA is associated with rapidly dividing cells, while PMP22 is associated with growth-arrested (non-dividing) cells. In aggregate, these three northern blots establish a correlation of EMP-1 mRNA expression with actively dividing cells, and suggest that the EMP-1 protein is needed for normal cell division.

Expression of recombinant rat EMP-1 protein in COS cells is shown in Figure 9. Recombinant human EMP-1 can be expressed as described in Example 6. EMP-1 protein is expressed in rat tissues that are making the mRNA, particularly those tissues in the digestive system. Within the digestive system, the EMP-1 protein is found to be expressed in the epithelial cells.

The striking homology of EMP-1 and PMP22 suggests possible functions for EMP-1. As indicated in the Background section, mutations in PMP22 lead to several important human diseases, most of which seem to be caused by alterations in the growth state of expressing cells, that is cells divide or fail to divide at inappropriate times. It is likely that EMP-1 will be involved in controlling the proliferative state of cells as well. Because EMP-1 is expressed in a wide variety of tissues, it is anticipated that mutations in EMP-1 or changes in the expression of EMP-1 may be associated with abnormal proliferation/differentiation of a wide range of tissues.

The elevated levels of EMP-1 expression in the intestinal tract suggests a role for EMP-1 in the proliferation and differentiation of cells in this region. The gastrointestinal tract is characterized by a continual and rapid renewal of its epithelial surface which continues throughout the animals life. Pluripotent stem cells anchored in the isthmus/neck regions of the gastric gland give rise to progeny displaying increased proliferation and reduced potentiality which progress to terminally differentiated mature cells (Gordon et al., Curr. Opin. Cell Biol. 6, 795-803 (1994)). During this differentiation process, the cells are highly migratory, with proliferation, migration and differentiation all being tightly coupled. EMP-1 is found mainly in the proliferation and differentiation zones of the outer gastric gland as well as in the mature epithelial cells of the gastric pit region. In these cells, EMP-1 appears to be associated with the plasma membrane, with no clear distinction between the basal, apical and lateral aspects.

### EMP-1 Nucleic acids

Isolated nucleic acid molecules encoding EMP-1 are encompassed by the invention. The molecules comprise sequences which encode EMP-1 as evidenced by generation of a recombinant protein of predicted size and post-translational modification similar to the endogenous protein. EMP-1 is preferably mammalian in origin and may be rat, mouse or human EMP-1 as shown in Figure 1, 2, and 3 (SEQ ID NOS: 1, 3 and 5) respectively. EMP-1 nucleic acids may also be variants of the sequences specifically disclosed, wherein⁻ a variant may be a naturally occurring alleleic variant or a substitution, deletion, or addition of one or more amino acids prepared by recombinant DNA techniques. The effects of mutation on EMP-1 biological activity may be predicted based upon corresponding mutation in related PMP22. PMP22 mutations leading to peripheral nervous system disorders are located in transmembrane domains which are highly conserved in EMP-1 (see Figure 4C). It is expected that mutations within these regions would also affect EMP-1 activity as well. Therefore, EMP-1 variants which retain biological activity are more likely to be found outside the conserved regions of the PMP/EMP family members shown in Figure 4C.

EMP-1 nucleic acids may be used as diagnostic reagents to study the structure of EMP-1 genes in biological samples. The molecules and reagents can be used to identify in a biological sample mutations or alterations in EMP-1 that may be predictive or diagnostic of pathological states, including cancer caused by abnormal proliferation of cells expressing EMP-1, and neurodegenerative disorders caused by an inability of cells expressing EMP-1 to maintain the normal physiology and differentiated state. The nucleic acid sequences can also be used to design oligonucleotide primers to look for mutations in EMP-1 that may be responsible for various pathological states. The method comprises incubating a biological sample having an altered EMP-1 nucleic acid sequence with a full-length or partial EMP-1 sequence; isolating the EMP-1 nucleic acid in the sample; and identifying the mutation or alteration. Particular tissues which would be most affected by abnormalities in EMP-1 include the digestive system, the nervous system, and the lung.

EMP nucleic acids may also be used to-identify related genes which are members of the PMP22/EMP-1 family. Nucleic acid probes can be made to conserved regions of EMP/PMP22 family members, such as the transmembrane domains, and used to screen cDNA or genomic libraries by hybridization or polymerase chain reaction (PCR) for related molecules.

Nucleic acids of the invention may also be used as reagents for gene therapy and anti-sense therapy to modify the expression of EMP-1 in selected tissues. EMP-1 expression may be increased by modifying tissue with an expression vector containing the EMP-1 coding region, wherein the vector produces EMP-1 in a tissue specific manner. Tissues that can be targeted for EMP-1 gene therapy include those associated with the digestive system, nervous system, lung and skin. Gene therapy is used to treat a variety of conditions including neurodegenerative diseases, lung disorders and gastrointestinal tract disorders. Endogenous EMP-1 expression may be decreased by using anti-sense nucleic acids to a portion of the EMP-1 coding region or to a control region operably linked thereto. The anti-sense nucleic acids may be the full-length EMP-1 gene or to a fragment thereof which hybridizes to the endogenous EMP-1 gene or a control region regulating expression of same. Anti-sense therapy is used to treat conditions resulting from overexpression of EMP-1 and include neurodegenerative diseases, cancer, lung disorders and gastrointestinal tract disorders.

Nucleic acid sequences of the invention are also used to produce recombinant EMP-1 as described below.

### Recombinant production of EMP-1

The invention provides for the recombinant production of EMP-1 in modified host cells. The invention provides materials for carrying out the invention, namely nucleic acid sequences encoding EMP-1, expression vectors containing EMP-1 sequences for producing protein, and modified host cells harboring EMP-1 expression vectors.

The nucleic acid molecules encoding EMP-1 are inserted into expression vectors and introduced into host cells using standard techniques. A suitable expression vector is any one which is capable of expressing EMP-1 in a host cell. It is preferred that an expression vector produces EMP-1 in a mammalian host cell. The modified host cells (i.e, those cells that contain EMP-1 nucleic acid sequences in an appropriate expression vector) are cultured under conditions which favor expression of EMP-1. The host cells of the invention may be any cells which support EMP-1 expression. In a preferred embodiment, the host cells will allow insertion of recombinant EMP-1 into the membrane in a functional configuration. A functional configuration of EMP-1 is characterized by proper insertion into the host cell membrane such that activation of EMP-1 occurs upon contacting a molecule capable of activation. An example of one such configuration is shown in Figure 4B. It is anticipated that the host cells will typically be mammalian cells. Preferably, host cells will be COS7, CHOd⁻, NIH 3T3, 293 or 32D cells.

Recombinant production of EMP-1 and display of the recombinant product on host cells are useful for evaluating candidate substances for their ability to bind to EMP-1 and effect a biological response upon formation of a complex with EMP-1. The host cells are used to screen for ligands of EMP-1. The host cells may also be used in screening procedures to identify peptide and small molecule effectors of EMP-1 and proteins which interact with EMP-1.

### Screening for EMP-1 agonists and antagonists

An EMP-1 agonist is defined as a substance or compound which stimulates the biological activity of EMP-1. An EMP-1 antagonist is defined as a substance or compound which decreases or inhibits the biological activity of EMP-1 in the presence of a stimulating compound. In general, screening procedures for EMP-1 agonists and antagonists involve contacting candidate substances with EMP-1 bearing host cells under conditions favorable for binding and measuring the extent of receptor activation (in the case of an agonist) or decrease in receptor activation (in the case of an antagonist).

EMP-1 activation may be measured in several ways. Typically, EMP-1 activation is apparent by a change in cell physiology such as an increase or decrease in growth rate, or by a change in differentiation state, or by a change in cell metabolism which can be detected in a microphysiometer.

### Antibodies

As described in Example 4, antibodies were generated to peptides from both the mouse and the rat extracellular domains. Antibodies to human EMP-1 may be generated by a similar procedure.

Antibodies specifically recognizing EMP-1 are encompassed by the invention. Antibodies are raised to the extracellular domain of EMP-1 using standard immunological techniques. The antigen may be the intact extracellular domain of membrane-bound EMP-1 or⁻ synthetic peptides comprising a portion of the extracellular domain. Antibodies may be polyclonal or monoclonal or may be produced recombinantly, such as for a humanized antibody. An antibody fragment which retains the ability to interact with EMP-1 is also provided. Such a fragment is produced by proteolytic cleavage of a full-length molecule or produced by recombinant DNA procedures. Antibodies of the invention are useful in diagnostic and therapeutic applications. They are used to detect and quantitate EMP-1 in biolgical samples, particuarly tissue samples. They may also be used to modulate the activity of EMP-1 by acting as an agonist or an antagonist.

The following examples are offered to more fully illustrate the invention, but are not construed as limiting the scope thereof.

### EXAMPLE 1

### Cloning and Sequencing of EMP-1 cDNAs

During a fetal rat intestine cDNA sequencing project, a 1003 base pair cDNA containing an open reading frame of 480 nucleotides was identified (Fig. 1). The predicted EMP-1 polypeptide of 160 amino acids residues has a calculated molecular weight of approximately 18 kDa. Computer-assisted analysis using the GCG software package reveals that amino acid residues 1-28, 64-89, 95-117 and 134-157 represent four hydrophobic, potentially membrane-spanning domains (Fig. 4C). The amino-terminal 16 amino acids have the characteristics of a signal peptide including a signal peptidase cleavage site after alanine-16 (Fig. 1). Furthermore, a single consensus sequence for N-linked glycosylation is present at asparagine-43 (Fig. 1, Fig. 4B).

### EXAMPLE 2

### Comparison of EMP-1 and PMP22 DNA Sequences

Comparison of the EMP-1 cDNA sequence to the GenBank database identified PMP22 as the closest known relative with a nucleotide identity of 58% over the open reading frame. Both the predicted EMP-1 protein and PMP22 are polypeptides of 160 amino acids which show 40% amino acid identity (Fig. 4A). The putative four membrane-spanning regions of EMP-1 and PMP22 are particularly well conserved. The first and second of these hydrophobic domains exhibit the highest degree of amino acid identity at 54% and 67%, respectively, while the third and fourth are only 30% and 37% identical.

Figure 4B depicts a theoretical model of the EMP-1 protein structure based on the hydrophobicity profile and the suggested structure of PMP22. Filled circles represent identical amino acid residues that are shared by rat EMP-1 and rat PMP22 while divergent residues are shown as open circles. The positions of the amino acids in PMP22 known to cause hereditary motor and sensory neuropathies (Suter et al., Hum. Mutation 3, 95-102 (1994) when mutated are highlighted in the EMP-1 sequence as diamonds (Fig. 4B). Interestingly, all of these mutations lie within the putative membrane-spanning domains and five of the six residues are conserved in rat EMP-1. The conservation of these amino acid residues suggests that they may be of functional significance.

Additional database searches with the EMP-1 and PMP22 sequences revealed that both display 30% amino acid identity to the lens fiber cell protein MP20 (Kumar et al. Exp. Eye Res. 56, 35-43 (1993)). MP20 is a 173 amino acid protein with similar structural features to EMP-1 and PMP22 (Fig. 4C). If MP20 is compared to PMP22 and EMP-1 simultaneously, the amino acid identity increases to 36% including strongly conserved motifs in the putative transmembrane domains (Fig. 4C).

### EXAMPLE 3

### In vitro Transcription and Translation of EMP-1

Both EMP-1 cDNA shown in Figure 1 and mouse PMP22 cDNA (Suter et al. Proc. Natl. Acad. Sci. USA 89, 4382-4386 (1992)) were cloned into the pcDNA-1 expression vector (Invitrogen) downstream of the bacteriophage T7 promoter region and used for *in vitro* transcription/translation assays . mRNA was produced from 0.5 µg of DNA with T7 polymerase and translated *in vitro* in the presence or absence of canine microsomal membranes (CMM) using a reticulocyte lysate system (TNT, Promega). The translation products were labelled metabolically by including [³⁵S]methionine in the reaction. The specificity of the reaction was tested using the vector pcDNA-1 as a control. The efficiency of the CMM was tested with the control reagents α-factor (glycosylation) and prolactin (signal peptide cleavage) according to the manufacturers instructions (TNT, Promega). One tenth of the translation product was denatured in 1% SDS and incubated for 4 hours at 37°C with 1 unit N-Glycosidase F in 50 mM sodium phosphate (pH 7.2), 12.5 mM EDTA, 2.5 mM sodium azide, 25% glycerol and 0.2% SDS. The proteins were separated by reducing 15% SDS PAGE and the gels were subsequently fixed 30 minutes in 50% methanol, 10% acetic acid and treated with enhancer (NEF-981G, Dupont). After drying the gels were exposed to X-ray film (RX, Fuji) overnight at -70°C.

The translation product of EMP-1 cDNA in the absence of canine microsomal membranes (CMM) has an apparent molecular weight of approximately 18 kDa which is in agreement with the calculated molecular weight of the EMP-1 protein. Translation in the presence of CMM results in a 4-6 kDa increase in molecular weight consistent with the presence of the single putative N-linked glycosylation site in the EMP-1 amino acid sequence (Fig. 5). This migrational shift can be reversed by deglycosylating the translation product with N-Glycosidase F (Fig. 5). The deglycosylated protein migrates identically to the unglycosylated EMP-1 protein suggesting that the putative N-terminal signal peptide is not removed during EMP-1 protein biosynthesis. A similar modification of PMP22 was seen with CMM confirming previous reports of endogenous PMP22 carrying an uncleaved signal peptide (Kitamura et al. in Proceedings of the 6th International Symposium on Glycoconjugates (Yamana, T. et al. eds) pp. 273-274, Jpn. Sci. Soc. Press, Tokyo (1981)).

### EXAMPLE 4

### Antibodies Reactive with Rat EMP-1

Anti-peptide antibodies were raised to synthetic peptides representing regions of the first and second putative extracellular loops of EMP-1: Amino acids are numbered according to the cDNA predicted polypeptide shown in Figure 1. A C-terminal cysteine residue was added to the loop1 peptide for coupling purposes. The peptides were coupled to keyhole limpet hemocyanin as described previously (Snipes et al. J. Cell Biol. 117, 225-238 (1992)). The conjugates were used to immunize New Zealand white rabbits with Freund's complete adjuvant and the animals were boosted 4 times with 500 µg peptide and incomplete adjuvant at 2 week intervals. Blood was taken from the animals and serum isolated. The activity of the immune serum was tested on the immunogen by solid-phase ELISA.

### EXAMPLE 5

### Expression Patterns of EMP-1 mRNA

A. Tissue distribution of EMP-1 and PMP22 mRNA.
   To elucidate the distribution of EMP-1 mRNA in the rat, EMP-1 cDNA (Fig. 1) was used to probe Northern blots of total RNA extracted from various tissues.
   RNA isolation and Northern blot analysis was carried out as follows. Total RNA was extracted from rat tissues using a modified acid phenol method. Briefly, tissues were homogenised into GT buffer (Chomczynski et al. Anal. Biochem. 162, 156-159 (1987)). The lysate was cleared and extracted twice with phenol/chloroform (1:1). The RNA was precipitated, resuspended in diethyl pyrocarbonate-treated H₂O and quantified by OD₂₆₀/OD₂₈₀ measurement. Ten µg of total RNA was loaded onto denaturing 1.2% agarose formaldehyde gels. Separated RNA was transferred to nylon membrane (Hybond N, Amersham) by capillarity and cross-linked with 240 m*J* of UV irradiation in a Stratalinker (Stratagene). Equal loading and transfer to the membrane was assessed by ethidium bromide staining. Membranes were prehybridized for 6 hours and hybridized 36 hours at 42°C in a solution containing 50% formamide. cDNA fragments of PMP22 and EMP-1 containing the entire open reading frames were labelled with ³²P-dCTP by random hexamer priming (Oligolabelling kit, Pharmacia). Northern blots were washed at high stringency and exposed to X-ray film (RX, Fuji) for 12 to 72 hours.
   EMP-1 transcripts can be found in all organs examined with the exception of the liver (Fig. 6a and b). The most prominent EMP-1 mRNA expression is observed in tail-derived skin and in the gastrointestinal tract. To examine a potential specific regional expression pattern, RNA was extracted from different regions of the gastrointestinal tract. Interestingly, we found that EMP-1 mRNA is not uniformly expressed throughout the stomach of the rat. The fundic region exhibits high levels of EMP-1 mRNA while expression in the corpus and pylorus are much lower. In the intestinal tract, the caecum and large intestine (colon and rectum) contain the highest levels of EMP-1 mRNA. EMP-1 transcripts are also detectable throughout the small intestine, but at far lower levels than in the fundus of the stomach, the caecum and large intestine. Considerable amounts of EMP-1 mRNA, similar to the expression in the duodenum, are also found in the brain and lung. Low-level EMP-1 expression is detectable in the heart, kidney, spleen, thymus and skeletal muscle.
   All tissues expressing EMP-1 mRNA contain 2.8 kb EMP-1 transcripts. In some regions of the gastrointestinal tract, however, including the fundus, ileum, caecum and colon, additional transcripts of approximately 1.7 kb hybridize with the EMP-1 cDNA (Fig. 6a). Prolonged washing of the blots at high stringency did not result in the preferential loss of one of the signals relative to the other, hence, we favour the interpretation that both the 2.8 kb and 1.7 kb transcripts are derived from the EMP-1 gene. Further studies of the differently sized transcripts will determine if they result from the use of alternative polyadenylation sites or arise by alternative splicing.
   The EMP-1-probed Northern blot was stripped and re-probed with labelled rat PMP22 cDNA (Fig. 6c and d). The results show that the tissue distribution of PMP22 mRNA and EMP-1 mRNA is similar, but that there are subtle differences in their relative expression levels. PMP22 and EMP-1 transcripts are co-expressed to high levels in the skin, fundus of the stomach, caecum, colon, rectum and duodenum. However, while the EMP-1 mRNA level is relatively high in colon compared to the rectum, PMP22 mRNA is low. Furthermore, PMP22 mRNA is more prominently expressed in the lung than EMP-1 mRNA but is relatively underrepresented in the brain (Fig. 6). Neither EMP-1 or PMP22 transcripts could be detected by Northern analysis of liver RNA.
B. Regulation of EMP-1 and PMP22 expression after sciatic nerve injury. The highest levels of PMP22 are found in myelinating Schwann cells of the PNS and expression is down regulated in the distal portion of the rat sciatic nerve following crush or cut injury Welcher et al. Proc. Natl. Acad. Sci. USA. 88, 7195-7199 (1991); Spreyer et al. EMBO J. 10, 3661-3668 (1991); Snipes et al. J. Cell Biol. 117, 225-238 (1992); De Leon et al. J. Neurosci. Res. 29, 437-448 (1991). EMP-1 mRNA levels in the sciatic nerve were examined under the same conditions. The sciatic nerves of male SIV rats (8-wk old; University of Zurich, Switzerland) were bilaterally exposed and cut unilaterally. Four days after injury, the degenerating distal portion of the traumatized nerve and, as a control, the undamaged contralateral nerve were removed and total RNA was isolated.
   Northern blot analysis reveals that EMP-1 transcripts are present at considerably lower levels in the adult sciatic nerve than PMP22 transcripts; Figure 7A represents exposures of 1 hour for PMP22 and approximately 36 hours for EMP-1. Furthermore, EMP-1 expression increases in the distal nerve following injury, in sharp contrast to PMP22 (Fig. 7A). These findings demonstrate that, although EMP-1 and PMP22 are coexpressed in PNS nerves, they appear to be differently regulated.
C. Regulation of EMP-1 and PMP22 expression in Schwann Cells in vitro. EMP-1 and PMP22 mRNA levels were compared in mitogen-expanded primary rat Schwann cells (pSC) and D6P2T Schwann cells grown in the presence or absence of forskolin. Forskolin has been shown to induce the expression of PMP22 and other myelin proteins in cultured Schwann cells via a mechanism proposed to partially mimic axon-Schwann cell interactions that occur during myelination Spreyer et al., supra; Pareek et al. J. Biol. Chem. 268, 10372-10379 (1993); Lemke et al. Development. 102, 499-504 (1988).
   Rat Schwann cells were isolated from the sciatic nerve of neonatal rats using the method of (Brockes et al Brain Res. 165, 105-118 (1979)) with modifications as described previously (Pareek et al., supra). The mitogen-expanded primary rat Schwann cells were cultured on poly-L-lysine coated culture plates in Dulbecco's Modified Eagles Medium (DMEM) containing 10% fetal calf serum (FCS) , 20 µg/ml crude glial growth factor, 5 µg/ml forskolin and 50 µg/ml gentamicin (Pareek et al., supra). The Schwann cell derived cell line, D6P2T (Bansal et al. *J*. Neurochem. 49, 1902-1911 (1987)) was grown in plastic culture dishes in DMEM containing 5% FCS and 50 µg/ml gentamicin. The cells were split into two groups and cultured in the same medium without forskolin. After 3 days, the cells had reached 70% confluency, one group of cells were treated with 20 µg/ml forskolin for 36 hours, while the second was maintained in the absence of forskolin. Subsequently, the cells were harvested in 5 M GT buffer and total RNA was extracted. PMP22 mRNA is upregulated by forskolin in pSC and D6P2T cells (Fig. 7B). In contrast, EMP-1 mRNA levels are decreased under the same conditions. This regulation is particularly prominent in the D6P2T cell line where EMP-1 mRNA is reduced in the presence of forskolin to barely detectable levels.
D. Regulation of EMP-1 and PMP22 after growth arrest in NIH3T3 Cells. NIH 3T3 fibroblasts were cultured and growth arrested by serum deprivation as described previously (Suter et al. J. Biol. Chem. 269, 1-14 (1994)). Exponentially growing and growth arrested cells were harvested into 5 M GT buffer. Under these conditions, NIH3T3 fibroblasts exhibited increased PMP22 mRNA expression (Fig. 7C). Manfioletti et al. Mol. Cell. Biol. 10, 2924-2930 (1990). In contrast to the regulation of PMP22, EMP-1 mRNA levels are strongly decreased under identical experimental conditions (Fig. 7C).
E. Expression of EMP-1 protein in the rat intestine. Rat tissues were homogenised into 8 M urea and cleared at 10,000 g for 10 min at 4°C. The protein concentration of the supernatant was assessed by Bradford assay and 50 µg of protein were denatured by heating to 95°C for 3 minutes in sample buffer containing 2% β-mercaptoethanol and loaded on a 12% SDS-PAGE. The proteins were electrotransferred to nitrocellulose membrane (Schleicher & Schuell) using a semi-dry blotter (Bio-Rad). Membranes were stained with Ponceau S to test transfer efficiency. Blots were blocked with 0.15% casein in phosphate buffered saline containing 0.2% Tween20 (Sigma), incubated with the polyclonal rabbit sera at a dilution of 1:500 in blocking buffer followed by a 1 hour incubation with Horse Radish Peroxidase-labelled goat anti-rabbit immunoglobulin (1:5000, Sigma). Detection was by chemiluminescence (ECL, Amersham) and exposure of X-ray film (RX, Fuji).
   Both anti-loop 1 and anti-loop 2 antibodies recognize a protein of approximately 25 kDa by Western blotting of various gastrointestinal tract tissue lysates (Fig. 8) but the anti-loopl antibodies are considerably more efficient. The labelling of immunoreactive proteins on Western blots can be blocked by preincubation of the antiserum in the presence of 250 µg/ml of immunogen peptide confirming specificity of the signal (Fig. 8A). Blocking is specific for the immunogen and is not effected by preincubation with the same concentration of a different peptide (Fig. 8A). The calculated molecular weight of the core EMP-1 protein is approximately 18 kDa which can be confirmed by *in vitro* transcription and translation of the cDNA (Fig. 5). The presence of a putative N-linked carbohydrate chain conceivably results in a protein with an apparent molecular weight of 25 kDa on reducing SDS-PAGE. However, additional post-translational modifications of the EMP-1 protein cannot be excluded.
   The most prominent expression of EMP-1 protein is seen in the stomach with lower levels being detectable in the caecum and large intestine. Expression in the duodenum and jejunum of the small intestine is considerably lower than in the other regions of the intestinal tract in accordance with the reduced mRNA levels found in these tissues (Fig. 6, 8A). Very low levels of the 25 kDa EMP-1 protein can also be detected in the lung (Fig. 8A), spleen and thymus.
   In addition to the 25 kDa protein, both EMP-1 antisera detect a similar array of larger proteins in the intestine (Fig. 8B). The presence of these additional immunoreactive species varies from experiment to experiment and between tissues. In general, the additional bands are most prominent in lysates containing higher amounts of EMP-1 protein. Since the two antisera are directed against independent regions of EMP-1 protein, these larger immunoreactive species are likely to represent aggregated molecules, a phenomenon frequently seen with highly hydrophobic proteins.
   Although the level of EMP-1 protein observed in some tissues does not strictly correlate with EMP-1 mRNA expression, EMP-1 protein can only be found in tissues where EMP-1 mRNA expression is seen. No immunoreactive proteins are detected by either antiserum in lysates of the EMP-1 mRNA-negative liver (Fig. 8A).

### EXAMPLE 6

### Expression of Recombinant EMP-1

A. Transient Expression of Recombinant Rat EMP-1.
   The EMP-1 cDNA shown in Figure 1 was subcloned into the EcoRV site of the pcDNA1 (InVitrogen) expression vector, down stream of the cytomegalovirus (CMV) promoter. The parent vector without cDNA insert was used in the negative control transfections. Recombinant DNA was purified by QIAGEN column isolation and quantitated by OD₂₆₀. COS cells exponentially growing in DMEM containing 10% FCS were trypsinised, washed in phosphate buffered saline and pelleted at 800 g. 1.5x10⁶ cells were resuspended in 200 µl phosphate buffered saline containing 5 µg of vector DNA. The cells were chilled on ice for 5 minutes, transferred to a 4 mm gap electropcration cuvette (Bio-Rad) and electroporated with 300 Volts and 125 µFarads. The transfected cells were chilled for 5 minutes on ice and split into seven 35 mm culture dishes containing 2 ml of DMEM and 10% FCS and cultured for 48 hours. The cells were then washed with Tris-buffered saline, fixed in DMEM containing 2% paraformaldehyde for 30 minutes, washed with Tris-buffered saline and permeabilized for 30 minutes in Tris-buffered saline containing 0.1% saponin. Unspecific binding sites were blocked for 30 minutes at room temperature in Tris-buffered saline, 2% bovine serum albumin, 0.1% porcine skin gelatin (type A Sigma), 2% goat serum and 0.1% saponin. The cells were incubated with the anti-EMP-1 antibodies diluted 1:500 in blocking buffer containing 0.02% saponin overnight at 4°C followed by FITC-labelled goat anti-rabbit immunoglobulin (Cappel) at 1:200 in blocking buffer with 0.02% saponin. After washing with Tris-buffered saline, coverslips were mounted using AF1 (Citifluor) and immunoreactivity visualised by confocal microscopy with a Bio-Rad, MRC-600 scanner in conjunction with a Zeiss Axiophot fluorescence microscope using Imaris image processing software (Bitplane AG, Technopark Zürich, Switzerland). Preimmune serum (1:500) as primary antiserum was used as a negative control.
   After COS cell transfection, both anti-loop 1 and anti-loop 2 antisera identified approximately 25% of the cells with strong immunoreactivity (Fig. 9). Neither antiserum nor preimmune sera recognised control COS cells transfected with the parental expression vector without EMP-1 insert. Since detection with anti-loop2 antiserum was more efficient than with the anti-loop1 antiserum, the former antibody was predominantly used in subsequent studies.
B. Expression of recombinant human EMP-1.
   The EMP-1 cDNA sequence as shown in Figure 3 was cloned into pCDNA-1 and transfected into COS cells. COS cells were cultured as in Example 6A and cell lysates were prepared and analyzed for EMP-1 protein expression by Western analysis. The protein is detected by anti-sera to human EMP-1.

### EXAMPLE 7

### Localization of EMP-1 Protein Expression in Rat Intestine

Figure 10a shows a schematic representation of tie topology of the rat gastric mucosa. The epithelial cells of the gastric pit are produced from stem cells in the isthmus/neck region of the gastric gland. These cells differentiate during their migration towards the gastric pit from where they are extruded (exfoliated) from the tip of the vilus (reviewed by Gordon and Hermiston Curr. Opin. Cell. Biol. 6, 795-803 (1994)). Transverse sections across the gastric pit show epithelial cells that are organized in circles around the intestinal lumen (Fig. 10a).

Tissue preparation and immunofluorescence were performed as follows. The stomach was removed from adult SIV rats, cut into blocks 10 mm by 5 mm, and snap frozen into CCT mounting medium (Tissue Tech) in isopentane on liquid nitrogen. Ten µm frozen sections were cut and thaw mounted onto slides subbed with 0.5% gelatin and 0.05% potassium chromate. The sections were fixed in 1% paraformaldehyde for 5 minutes and washed in phosphate buffered saline. Unspecific binding sites were blocked for 1 hour with goat serum diluted 1:50 in phosphate buffered saline. Sections were incubated overnight at 4°C or 4 hours at room temperature with 1:500 dilution of primary antiserum in phosphate buffered saline containing 0.2% Tween20 followed by 2 hours with 1:200 dilution of Texas Red-labelled donkey anti-rabbit immunoglobulin (Jackson Immunoresearch Laboratories). After extensive washing in phosphate buffered saline containing 0.2% Tween20, sections were coated with AF1 (Citifluor) and cover-slips mounted. Immune reactivity was visualised by high resolution confocal microscopy. Preimmune serum (1:500) as primary antiserum was used as a negative control.

Ten µm frozen sections of corpus gastricum were stained with anti-loop2 antiserum. Strong immunoreactivity was detected in the outer epithelial cells of the gastric mucosa from the tip of the vilus down towards the isthmus and neck of the gastric gland (Fig. 10b, c). In transverse section, the EMP-1 immunoreactivity appears to be associated with the plasmamembrane of epithelial cells in the gastric pits (Fig. 10e). The epithelial cells deeper in the gastric gland show little or no immunoreactivity and specific labelling was not detectable in the base of the gastric gland or in the sub-mucosal muscle layer (Fig. 10b).

## Claims

1. An isolated nucleic acid molecule encoding a protein having the amino acid sequence shown in Figures 1 or 3, or a naturally occurring allelic variant thereof.

2. The molecule of claim 1 which encodes a mammalian epithelial membrane protein.

3. The molecule of claim 1 which encodes a rat epithelial membrane protein having the sequence shown in Figure 1.

4. An isolated nucleic acid sequence which encodes a mouse epithelial membrane protein having the sequence shown in Figure 2.

5. The molecule of claim 1 which encodes a human epithelial membrane protein having the sequence shown in Figure 3.

6. A DNA molecule comprising an expression system which, when transfected into a host cell, is capable of expressing a protein having the amino acid sequence shown in Figures 1 or 3, or a naturally occurring allelic variant thereof.

7. The DNA molecule of claim 6 wherein the expression system comprises a nucleotide sequence encoding a protein having the amino acid sequence shown in Figures 1 or 3, or a naturally occurring allelic variant thereof.

8. A host cell modified with the DNA molecule of claim 6.

9. The host cell of claim 8 which is a mammalian cell.

10. The host cell of clain 8 wherein said encoded protein is displayed at the cell surface.

11. An antibody or fragment thereof which specifically binds to a protein having the amino acid sequence shown in Figures 1 or 3, or a naturally occurring allelic variant thereof.

12. The antibody of claim 11 which is an agonist of a protein having the amino acid sequence shown in Figures 1 or 3, or a naturally occurring allelic variant thereof.

13. The antibody of claim 11 which is an antagonist of a protein having the amino acid sequence shown in Figures 1 or 3, or a naturally occurring allelic variant thereof.

14. A method of expressing a protein having the amino acid sequence shown in Figures 1 or 3, or a naturally occurring allelic variant thereof, in a host cell comprising:
introducing the DNA molecule of claim 6 into the cells; and
culturing the cells under conditions which allow the expression of a protein having the amino acid sequence shown in Figures 1 or 3, or a naturally occurring allelic variant thereof.

15. The method of claim 14 wherein the expression protein is displayed at the surface of the host cell.

16. A method of altering the expression of an endogenous gene encoding an epithelial membrane protein having the amino acid sequence shown in Figures 1, 2 or 3 in a selected tissue *in vitro* comprising modifying the tissue with an exogenous nucleic acid sequence encoding an epithelial membrane protein having the amino acid sequence shown in Figures 1, 2 or 3 wherein the exogenous sequence increases or decreases the level of epithelial membrane protein in said tissue.

17. The method of a claim 16 wherein the exogenous nucleic acid sequence contains the epithelial membrane protein coding region operably linked to a tissue-specific expression system.

18. The method of claim 16 wherein the exogenous nucleic acid sequence is complementary to a portion of the endogenous gene encoding an epithelial membrane protein having the amino acid sequence shown in Figures 1, 2 or 3.

19. A method to assess the ability of a candidate substance to behave as an agonist of an epithelial membrane protein having the amino acid sequence shown in Figures 1, 2 or 3, which method comprises:
incubating the cells of claim 10 with the candidate substance under conditions which allow activation of an epithelial membrane protein having the amino acid sequence shown in Figures 1, 2 or 3; and
measuring the activation of said epithelial membrane protein resulting therefrom.

20. A method to assess the ability of a candidate substance to behave as an antagonist to an epithelial membrane protein having the amino acid sequence shown in Figures 1, 2 or 3, which method comprises:
incubating the cells of claim 10 with the candidate substance wherein an epithelial membrane protein having the amino acid sequence shown in Figures 1, 2 or 3 has been activated; and
measuring a subsequent decrease in activation of said epithelial membrane protein.

21. A method for detecting the presence of a protein having activity of an epithelial membrane protein having the amino acid sequence shown in Figures 1, 2 or 3, in a biological sample comprising:
incubating the sample with the antibody of claim 11 under conditions allowing binding of the antibody to an epithelial membrane protein; and
detecting the bound antibody.

22. A method for identifying in a biological sample mutations or alterations in a nucleic acid sequence comprising:
incubating the sample with the nucleic acid of claim 1 or a portion thereof,
under conditions permitting hybridazation;
isolating the nucleic acid in the sample; and
identifying the mutation or alteration in the nucleic acid.

23. A DNA molecule comprising an expression system which, when transfected into a host cell, is capable of expressing a protein having the amino acid sequence shown in Figure 2.

24. The DNA molecule of claim 23 wherein the expression system comprises a nucleotide sequence encoding a protein having the amino acid sequence shown in Figure 2.

25. An antibody or fragment thereof which specifically binds to a protein having the amino acid sequence shown in Figure 2.

26. The antibody of claim 25 which is an agonist of a protein having the amino acid sequence shown in Figure 2.

27. The antibody of claim 25 which is an antagonist of a protein having the amino acid sequence shown in Figure 2.

## Patentansprüche

1. Isoliertes Nukleinsäuremolekül, das für ein Protein mit der in den Figuren 1 oder 3 gezeigten Aminosäuresequenz oder für eine natürlich vorkommende allele Variante davon kodiert.

2. Molekül gemäß Anspruch 1, das für ein Säuger-Epithel-Membranprotein kodiert.

3. Molekül gemäß Anspruch 1, das für ein Ratten-Epithel-Membranprotein mit der in Figur 1 gezeigten Sequenz kodiert.

4. Isolierte Nukleinsäuresequenz, die für ein Mäuse-Epithel-Membranprotein mit der in Figur 2 gezeigten Sequenz kodiert.

5. Molekül gemäß Anspruch 1, das für ein menschliches Epithel-Membranprotein mit der in Figur 3 gezeigten Sequenz kodiert.

6. DNA-Molekül, umfassend ein Expressionssystem, das bei einer Transfektion in eine Wirtszelle ein Protein mit der in den Figuren 1 oder 3 gezeigten Aminosäuresequenz oder eine natürlich vorkommende allele Variante davon exprimieren kann.

7. DNA-Molekül gemäß Anspruch 6, wobei das Expressionssystem eine Nukleotidsequenz umfasst, die für ein Protein mit der in den Figuren 1 oder 3 gezeigten Aminosäuresequenz oder eine natürlich vorkommende allele Variante davon kodiert.

8. Wirtszelle, die mit dem DNA-Molekül gemäß Anspruch 6 modifiziert ist.

9. Wirtszelle gemäß Anspruch 8, die eine Säugerzelle ist.

10. Wirtszelle gemäß Anspruch 8, wobei das kodierte Protein auf der Zelloberfläche präsentiert wird.

11. Antikörper oder Fragment davon, der spezifisch an ein Protein mit der in den Figuren 1 oder 3 gezeigten Aminosäuresequenz oder an eine natürlich vorkommende allele Variante davon bindet.

12. Antikörper gemäß Anspruch 11, der ein Agonist eines Proteins mit der in den Figuren 1 oder 3 gezeigten Aminosäuresequenz oder einer natürlich vorkommenden allelen Variante davon ist.

13. Antikörper gemäß Anspruch 11, der ein Antagonist eines Proteins mit der in den Figuren 1 oder 3 gezeigten Aminosäuresequenz oder einer natürlich vorkommenden allelen Variante davon ist.

14. Verfahren zur Expression eines Proteins mit der in den Figuren 1 oder 3 gezeigten Aminosäuresequenz oder einer natürlich vorkommenden allelen Variante davon in einer Wirtszelle, umfassend:
Einbringen des DNA-Moleküls gemäß Anspruch 6 in die Zellen; und
Kultivieren der Zellen unter Bedingungen, die die Expression eines Proteins mit der in den Figuren 1 oder 3 gezeigten Aminosäuresequenz oder einer natürlich vorkommenden allelen Variante davon erlauben.

15. Verfahren gemäß Anspruch 14, wobei das Expressionsprotein auf der Oberfläche der Wirtszelle präsentiert wird.

16. Verfahren zur Veränderung der Expression eines endogenen Gens, das für ein Epithel-Membranprotein mit der in den Figuren 1, 2 oder 3 gezeigten Aminosäuresequenz kodiert, in einem ausgewählten Gewebe *in vitro,* umfassend die Modifizierung des Gewebes mit einer exogenen Nukleinsäuresequenz, die für ein Epithel-Membranprotein mit der in den Figuren 1, 2 oder 3 gezeigten Aminosäuresequenz kodiert, wobei die exogene Sequenz die Menge von Epithel-Membranprotein in dem Gewebe erhöht oder verringert.

17. Verfahren gemäß Anspruch 16, wobei die exogene Nukleinsäuresequenz die für das Epithel-Membranprotein kodierende Region in funktioneller Verbindung mit einem gewebespezifischen Expressionssystem enthält.

18. Verfahren gemäß Anspruch 16, wobei die exogene Nukleinsäuresequenz zu einem Teil des endogenen Gens komplementär ist, das für ein Epithel-Membranprotein mit der in den Figuren 1, 2 oder 3 gezeigten Aminosäuresequenz kodiert.

19. Verfahren zur Bewertung der Fähigkeit einer Kandidatensubstanz, sich als Agonist eines Epithel-Membranproteins mit der in den Figuren 1, 2 oder 3 gezeigten Aminosäuresequenz zu verhalten, umfassend:
Inkubation der Zellen gemäß Anspruch 10 mit der Kandidatensubstanz unter Bedingungen, die eine Aktivierung eines Epithei-Membranproteins mit der in den Figuren 1, 2 oder 3 gezeigten Aminosäuresequenz erlauben; und
Messen der Aktivierung des Epithei-Membranproteins, die sich daraus ergibt.

20. Verfahren zur Bewertung der Fähigkeit einer Kandidatensubstanz, sich als Antagonist eines Epithel-Membranproteins mit der in den Figuren 1, 2 oder 3 gezeigten Aminosäuresequenz zu verhalten, umfassend:
Inkubation der Zellen gemäß Anspruch 10 mit der Kandidatensubstanz, wobei ein Epithel-Membranprotein mit der in den Figuren 1, 2 oder 3 gezeigten Aminosäuresequenz aktiviert wurde; und
Messen einer darauffolgenden Verringerung der Aktivierung des Epithel-Membranproteins.

21. Verfahren zum Nachweis eines Proteins mit der Aktivität eines Epithel-Membranproteins mit der in den Figuren 1, 2 oder 3 gezeigten Aminosäuresequenz in einer biologischen Probe, umfassend:
Inkubation der Probe mit dem Antikörper gemäß Anspruch 11 unter Bedingungen, die eine Bindung des Antikörpers an ein Epithel-Membranprotein erlauben; und
Nachweis des gebundenen Antikörpers.

22. Verfahren zur Identifizierung von Mutationen oder Veränderungen einer Nukleinsäuresequenz in einer biologischen Probe, umfassend:
Inkubation der Probe mit der Nukleinsäure gemäß Anspruch 1 oder einem Teil davon unter Bedingungen, die eine Hybridisierung ermöglichen;
Isolierung der Nukleinsäure in der Probe; und.
Identifizierung der Mutation oder Veränderung in der Nukleinsäure.

23. DNA-Molekül, umfassend ein Expressionssystem, das bei einer Transfektion in eine Wirtszelle fähig zur Expression eines Proteins mit der in Figur 2 gezeigten Aminosäuresequenz ist.

24. DNA-Molekül gemäß Anspruch 23, wobei das Expressionssystem eine Nukleotidsequenz umfasst, die für ein Protein mit der in Figur 2 gezeigten Aminosäuresequenz kodiert.

25. Antikörper oder Fragment davon, der spezifisch an ein Protein mit der in Figur 2 gezeigten Aminosäuresequenz bindet.

26. Antikörper gemäß Anspruch 25, der ein Agonist eines Proteins mit der in Figur 2 gezeigten Aminosäuresequenz ist.

27. Antikörper gemäß Anspruch 25, der ein Antagonist eines Proteins mit der in Figur 2 gezeigten Aminosäuresequenz ist.

## Revendications

1. Molécule d'acide nucléique isolée codant pour une protéine ayant la séquence d'acides aminés représentée dans les figures 1 à 3, ou un variant allélique naturel de celle-ci.

2. Molécule selon la revendication 1, qui code pour une protéine de membrane épithéliale mammalienne.

3. Molécule selon la revendication 1, qui code pour une protéine de membrane épithéliale de rat ayant la séquence représentée dans la figure 1.

4. Séquence d'acide nucléique isolée qui code pour une protéine de membrane épithéliale de souris ayant la séquence représentée dans la figure 2.

5. Molécule selon la revendication 1 qui code pour une protéine de membrane épithéliale humaine ayant la séquence représentée dans la figure 3.

6. Molécule d'ADN comprenant un système d'expression qui lors de la transfection dans une cellule hôte, est capable d'exprimer une protéine ayant la séquence d'acides aminés représentée dans les figures 1 ou 3, ou un variant allélique naturel de celle-ci.

7. Molécule d'ADN selon la revendication 6, dans laquelle le système d'expression comprend une séquence nucléotidique codant pour une protéine ayant la séquence d'acides aminés représentée dans les figures 1 ou 3, ou un variant allélique naturel de celle-ci.

8. Cellule hôte modifiée avec la molécule d'ADN selon la revendication 6.

9. Cellule hôte selon la revendication 8, qui est une cellule mammalienne.

10. Cellule hôte selon la revendication 8, dans laquelle ladite protéine codée est exposée à la surface des cellules.

11. Anticorps ou fragment de celui-ci qui se lie spécifiquement à une protéine ayant la séquence d'acides aminés représentée dans les figures 1 ou 3, ou un variant allélique naturel de celle-ci.

12. Anticorps selon la revendication 11, qui est un agoniste d'une protéine ayant la séquence d'acides aminés représentée dans les figures 1 ou 3, ou un variant allélique naturel de celle-ci.

13. Anticorps selon la revendication 11, qui est un antagoniste d'une protéine ayant la séquence d'acides aminés représentée dans les figures 1 ou 3, ou un variant allélique naturel de celle-ci.

14. Procédé pour l'expression d'une protéine ayant la séquence d'acides aminés représentée dans les figures 1 ou 3, ou d'un variant allélique naturel de celle-ci, dans une cellule hôte comprenant:
l'introduction de la molécule d'ADN selon la revendication 6 dans les cellules; et
la culture des cellules dans des conditions qui permettent l'expression d'une protéine ayant la séquence d'acides aminés représentée dans les figures 1 ou 3, ou d'un variant allélique naturel de celle-ci.

15. Procédé selon la revendication 14, dans lequel la protéine d'expression est exposée à la surface de la cellule hôte.

16. Procédé pour modifier l'expression d'un gène endogène codant pour une protéine de membrane épithéliale ayant la séquence d'acides aminés représentée dans les figures 1, 2 ou 3 dans un tissu sélectionné *in vitro*, comprenant la modification du tissu avec une séquence d'acide nucléique exogène codant pour une protéine de membrane épithéliale ayant la séquence d'acides aminés représentée dans les figures 1, 2 ou 3, tandis que la séquence exogène augmente ou diminue la teneur en protéine de membrane épithéliale dans ledit tissu.

17. Procédé selon la revendication 16, dans lequel la séquence d'acide nucléique exogène contient la région codante de protéine de membrane épithéliale liée de façon utilisable à un système d'expression spécifique d'un tissu.

18. Procédé selon la revendication 16, dans lequel la séquence d'acide nucléique exogène est complémentaire d'une portion du gène endogène codant pour une protéine de membrane épithéliale ayant la séquence d'acides aminés représentée dans les figures 1, 2 ou 3.

19. Procédé pour évaluer l'aptitude d'une substance candidate à se comporter comme un agoniste d'une protéine de membrane épithéliale ayant la séquence d'acides aminés représentée dans les figures 1, 2 ou 3, lequel procédé comprend:
l'incubation des cellules selon la revendication 10 avec la substance candidate dans des conditions qui permettent l'activation d'une protéine de membrane épithéliale ayant la séquence d'acides aminés représentée dans les figures 1,2 ou 3; et
la mesure de l'activation de ladite protéine de membrane épithéliale qui en résulte.

20. Procédé pour évaluer l'aptitude d'une substance candidate à se comporter comme un antagoniste d'une protéine de membrane épithéliale ayant la séquence d'acides aminés représentée dans les figures 1, 2 ou 3, lequel procédé comprend:
l'incubation des cellules selon la revendication 10 avec la substance candidate dans laquelle une protéine de membrane épithéliale ayant la séquence d'acides aminés représentée dans les figures 1, 2 ou 3 a été activée; et
la mesure d'une diminution subséquente d'activation de ladite protéine de membrane épithéliale.

21. Procédé pour la détection de la présence d'une protéine ayant l'activité d'une protéine de membrane épithéliale ayant la séquence d'acides aminés représentée dans les figures 1, 2 ou 3, dans un échantillon biologique, comprenant:
l'incubation de l'échantillon avec l'anticorps selon la revendication 11 dans des conditions permettant la liaison de l'anticorps à une protéine de membrane épithéliale; et
la détection de l'anticorps lié.

22. Procédé pour l'identification dans un échantillon biologique de mutations ou de modifications dans une séquence d'acide nucléique, comprenant:
l'incubation de l'échantillon avec l'acide nucléique selon la revendication 1 ou une portion de celui-ci, dans des conditions permettant l'hybridation;
l'isolement de l'acide nucléique dans l'échantillon; et
l'identification de la mutation ou la modification dans l'acide nucléique.

23. Molécule d'ADN comprenant un système d'expression qui, lors d'une transfection dans une cellule hôte, est capable d'exprimer une protéine ayant la séquence d'acides aminés représentée dans la figure 2.

24. Molécule d'ADN selon la revendication 23, dans laquelle le système d'expression comprend une séquence nucléotidique codant pour une protéine ayant la séquence d'acides aminés représentée dans la figure 2.

25. Anticorps ou fragment de celui-ci, qui se lie spécifiquement à une protéine ayant la séquence d'acides aminés représentée dans la figure 2.

26. Anticorps selon la revendication 25, qui est un agoniste d'une protéine ayant la séquence d'acides aminés représentée dans la figure 2.

27. Anticorps selon la revendication 25, qui est un antagoniste d'une protéine ayant la séquence d'acides aminés représentée dans la figure 2.


### SEQUENCE LISTING

(1) GENERAL INFORMATION:
   (i) APPLICANT: Amgen Inc.
   (ii) TITLE OF INVENTION: EPITHELIAL MEMBRANE PROTEIN-1
   (iii) NUMBER OF SEQUENCES: 7
   (iv) CORRESPONDENCE ADDRESS:
      (A) ADDRESSEE: Amgen Inc.
      (B) STREET: 1840 Dehavilland Drive
      (C) CITY: Thousand Oaks
      (D) STATE: California
      (E) COUNTRY: USA
      (F) ZIP: 91320-1789
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: 08/563,839
      (B) FILING DATE: 23-NOV-1995
      (C) CLASSIFICATION:
   (viii) ATTORNEY/AGENT INFORMATION:
      (A) NAME: Winter, Robert B.
      (C) REFERENCE/DOCKET NUMBER: A-366
(2) INFORMATION FOR SEQ ID NO:1:
   (i) SEQUENCE CHARACTERISTICS:
      (A) LENGTH: 1003 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 132..611
   (xi) SEQUENCE DESCRIPTION: SEQ ID NO:1:
   GTCGACCCAC GCGTCCGAAA CCTCCTGAAG AGAGGACCAG ACCAGCAGCC AGCGCCACCA      60
   CTCAGGGCAT CTGCCTCTGT CACTGGATAC TCCAGAATTC TCTACTCAGG AGTTACAAAA     120
   AAGAAGCCAA G ATG TTG GTG CTA CTG GCC GGT CTC TTC GTG GTC CAC ATC       170
                Met Leu Val Leu Leu Ala Gly Leu Phe Val Val His Ile
                 1               5                  10
   GCC ACT GCC ATT ATG CTG TTT GTC TCC ACC ATT GCC AAC GTC TGG ATG        218
   Ala Thr Ala Ile Met Leu Phe Val Ser Thr Ile Ala Asn Val Trp Met
        15                  20                  25
   GTG GCG GAC GGT ATA GAC TCG TCC ATA GGG CTT TGG AAG AAC TGC ACC        266
   Val Ala Asp Gly Ile Asp Ser Ser Ile Gly Leu Trp Lys Asn Cys Thr
    30                  35                  40                  45
   AGT GGC AGC TGT GAC GGC TCT CTG AGC TAC GGC AAT GAT GAT GCT ATC        314
   Ser Gly Ser Cys Asp Gly Ser Leu Ser Tyr Gly Asn Asp Asp Ala Ile
                    50                  55                  60
   AAG GCA GTG CAA GCT TTC ATG ATC CTC TCC ATC ATC TTC TCT ATA ATC        362
   Lys Ala Val Gln Ala Phe Met Ile Leu Ser Ile Ile Phe Ser Ile Ile
                65                  70                  75
   TCC CTC GTG GTC TTC GTG TTC CAG CTC TTC ACC ATG GAG AAG GGA AAC        410
   Ser Leu Val Val Phe Val Phe Gln Leu Phe Thr Met Glu Lys Gly Asn
            80                  85                  90
   CGG TTC TTC CTC TCG GGA TCC ACC ATG CTG GTG TGC TGG CTG TGC ATT        458
   Arg Phe Phe Leu Ser Gly Ser Thr Met Leu Val Cys Trp Leu Cys Ile
        95                 100                 105
   CTG ATT GGA GTG TCT ATC TAC ACT CAC CAC TAC GCC CAC AGC GAA GGG        506
   Leu Ile Gly Val Ser Ile Tyr Thr His His Tyr Ala His Ser Glu Gly
   110                 115                 120                 125
   AAC TTT TTC CCC AGC AGC CAT CAA GGC TAC TGT TTC ATC CTG ACC TGG        554
   Asn Phe Phe Pro Ser Ser His Gln Gly Tyr Cys Phe Ile Leu Thr Trp
                   130                 135                 140
   ATT TGC TTC TGC TTC AGC TTC ATC ATC GGC ATA CTC TAT ATG GTC CTG        602
   Ile Cys Phe Cys Phe Ser Phe Ile Ile Gly Ile Leu Tyr Met Val Leu
               145                 150                 155
   AGG AAG AAA TAAGCTCGTG GGCATCTGGG GTGGGGGTGG GGAAGTAGGG                651
   Arg Lys Lys
           160
   AGGAGGAAGC AACTGAATCC GGGAGGGAAG CAGAAGTCAC TGTGTAGGGA TAACCAAGGG      711
   AGGGGAGGGG GGAAGGGAGG GGGAAAGGAA GAGTAGGAGA GGCCCAAACC CAAACCATAT      771
   CTGGGGGGGC GTGGTTCTCT ACTGCCAAAC GCCCATCCTT GGAAGAAAGT TGTTGGCTAC      831
   TATGCTGATG CTTCCTTGAG GCCACCAGAG AGTCCTCCTC TAGCCACCAA ATATGGCCCC      891
   ATCTATCCTC AATTACCGAC ACTTGGGGCC TCACCAGCTG CCATTCCACT GGCGCCACTC      951
   TTGAGGGTGA CTGCTGGGTC ATACACTGAG GTCTTGCAAA CCCATTCGTG TA             1003
(2) INFORMATION FOR SEQ ID NO:2:
   (i) SEQUENCE CHARACTERISTICS:
      (A) LENGTH: 160 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Leu Val Leu Leu Ala Gly Leu Phe Val Val His Ile Ala Thr Ala
    1               5                  10                  15
   Ile Met Leu Phe Val Ser Thr Ile Ala Asn Val Trp Met Val Ala Asp
               20                  25                  30
   Gly Ile Asp Ser Ser Ile Gly Leu Trp Lys Asn Cys Thr Ser Gly Ser
           35                  40                  45
   Cys Asp Gly Ser Leu Ser Tyr Gly Asn Asp Asp Ala Ile Lys Ala Val
       50                  55                  60
   Gln Ala Phe Met Ile Leu Ser Ile Ile Phe Ser Ile Ile Ser Leu Val
   65                  70                  75                  80
   Val Phe Val Phe Gln Leu Phe Thr Met Glu Lys Gly Asn Arg Phe Phe
                   85                  90                  95
   Leu Ser Gly Ser Thr Met Leu Val Cys Trp Leu Cys Ile Leu Ile Gly
               100                 105                 110
   Val Ser Ile Tyr Thr His His Tyr Ala His Ser Glu Gly Asn Phe Phe
           115                 120                 125
   Pro Ser Ser His Gln Gly Tyr Cys Phe Ile Leu Thr Trp Ile Cys Phe
       130                 135                 140
   Cys Phe Ser Phe Ile Ile Gly Ile Leu Tyr Met Val Leu Arg Lys Lys
   145                 150                 155                 160
(2) INFORMATION FOR SEQ ID NO:3:
   (i) SEQUENCE CHARACTERISTICS:
      (A) LENGTH: 2323 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 183..662
   (xi) SEQUENCE DESCRIPTION: SEQ ID NO:3:
   ACACACCCCA GCACATCAGC CCAGGAAACT TATAACCTCG GGAGTCAGGT CCCTCCCCTC      60
   ACTGTGGTTG CAAATCTCCT GAAGAGAGGA CCAGACCAGC AGCCTGCTCT ACCACCCAGG     120
   GCATCTGCCT CTCTCACTGG ATACTCCAGA ATTCTCTACT CAGAAGTCAC CAAAAAGCCA     180
   AG ATG TTG GTG CTA CTG GCT GGT CTC TTT GTG GTC CAC ATT GCC ACT        227
      Met Leu Val Leu Leu Ala Gly Leu Phe Val Val His Ile Ala Thr
       1               5                  10                  15
   GCC ATT ATG CTG TTT GTC TCC ACC ATT GCC AAC GTC TGG ATG GTT GCA       275
   Ala Ile Met Leu Phe Val Ser Thr Ile Ala Asn Val Trp Met Val Ala
                   20                  25                  30
   GAT TAC GCA AAT GCA TCT GTA GGG CTT TGG AAG AAC TGC ACT GGT GGT       323
   Asp Tyr Ala Asn Ala Ser Val Gly Leu Trp Lys Asn Cys Thr Gly Gly
               35                  40                  45
   AAC TGC GAC GGC TCC CTG TCC TAC GGC AAT GAA GAT GCT ATC AAG GCA       371
   Asn Cys Asp Gly Ser Leu Ser Tyr Gly Asn Glu Asp Ala Ile Lys Ala
           50                  55                  60
   GTG CAA GCC TTC ATG ATC CTC TCC ATC ATC TTC TCC ATC ATC TCC CTC       419
   Val Gln Ala Phe Met Ile Leu Ser Ile Ile Phe Ser Ile Ile Ser Leu
       65                  70                  75
   GTG GTC TTC GTG TTC CAG CTC TTC ACT ATG GAG AAG GGA AAC CGG TTC       467
   Val Val Phe Val Phe Gln Leu Phe Thr Met Glu Lys Gly Asn Arg Phe
   80                  85                  90                  95
   TTC CTC TCG GGG TCC ACC ATG CTG GTG TGC TGG CTG TGT ATC CTG GTT       515
   Phe Leu Ser Gly Ser Thr Met Leu Val Cys Trp Leu Cys Ile Leu Val
                   100                 105                 110
   GGA GTG TCA ATC TAC ACT CAT CAT TAC GCC CAC AGC GAA GGG AAC TTC       563
   Gly Val Ser Ile Tyr Thr His His Tyr Ala His Ser Glu Gly Asn Phe
               115                 120                 125
   AAC TCC AGC AGC CAC CAA GGC TAT TGT TTC ATC CTG ACC TGG ATC TGC       611
   Asn Ser Ser Ser His Gln Gly Tyr Cys Phe Ile Leu Thr Trp Ile Cys
           130                 135                 140
   TTC TGT TTC AGC TTC ATC ATC GGC ATA CTC TAT ATG GTC CTG AGG AAG       659
   Phe Cys Phe Ser Phe Ile Ile Gly Ile Leu Tyr Met Val Leu Arg Lys
       145                 150                 155
   AAA TAAGCCGAAT ACGCTCATGG GCGTCTGGGG GCGGGGTGGG CTGGGTAGGA            712
   Lys
   160
   GGAAGCAACC TAACCTGGGA GGGAAGCAGG AGTCACTGTG TAGGAATAAC AGAGAGGGGA     772
   GGGGGGTGGG GAGAGGGAAG GAAGAGGGGG AGAGGCCCAA ACCCAAACCA TATCTGGGGC     832
   GGTGGGATTC TCTACTGCCA AGCACCCATC CTTGGAAGAA AGTTGTTGGC TGATATGCTG     892
   ATGCTTCCTT GACGTCACCA GAGAGTCCTC CTCTAGCCAC CAAATATGGC CCCGTCCATC     952
   CTCAATTACA TACACTCGGG GCCTCCCCAG CTGCCATACC ACTGGCGCCA CTCTTGAGGG    1012
   TGGCTGCTGG GTCACACACT GAGGTCTTCC ACATCCCATA TCATCAAGTT CTGATGGTGG    1072
   TTCAGGTCTT AGCAAGAGCA GATATTGCTC GATGCTGAGG CTAAGTCTGG AAGCCACTTT    1132
   GTCCTTGTGA CCTAAAACCA AACATCAAAT CCAGATCCCA TGTGCCTGTA GTGGGAGCTT    1192
   TGGCCAGGAA GCCAATGTGC ATATTTGGTG GCCTTTCTAA CAAAAGTATA GGATGATGAG    1252
   AGATGGTTTG TAAGTTCAAA GCTGATGGAA TTGGTTTAGC CAAGAAATGG AAGTTTCTAC    1312
   CCCAGAGGAT CCTTGGAGAC AGGTGGGGAC AGGCAGTGCT CCTCAGTCAC GTGTCACCGA    1372
   GCTGTCCCTC ATGGAGGCCT CCTGTTGTGA ACTCTGCTAG ACTCTCACTT ACAGCCAAGG    1432
   CAGCTTTTCT GGAGTTTTTC TTAGATTCTC TAGAGCCAAA GATGATAATG CCTCACAAAA    1492
   CATAGGGTCA AAGCATATGC CCACCGCAGT GCTATAGTAA GTTTGTGGGT TTTTAGGATT    1552
   CCCCCAAAGC ACTCAATGTA TCTTGTATAT GTAACAGGGG AGAAATGCAT GTGTTCCTTT    1612
   GACATACAAT TCTGAACTAG GAATATTTGA GGAAGTCCAA TGATGACCAA CAACACTGGG    1672
   GACCAGAATA TAACATCTAA ATGCAGTAGT CACTGTTGCT TTGACCTGGG CTGGAGTGGT    1732
   CTCCTCTCAA CAGCTTTCAT CACACTATTT TCCAGCTAAA GATGGCAAAG CTGTAAGCCA    1792
   ATTAACATAT ACACCAACCT AAACTAAAGA ACCAGTCCTG AGGGTGTGAG AAAGGTGCTA    1852
   TCTGGTTATG GATTATTAAG CAAACCATAT TTCATTTATG TTGAGAAGAG AATGCCTGCC    1912
   CTCAGGGAAA AAAAAATGTA ATTGTGTGAG ATGAATAAAG TCCTGGTGAT AGGCAGACAG    1972
   TTTCTTTTTT AAAACAGGAG AAACTCTTAG GGCATCCAGA CAGATGGTAG CTAAATTGTT    2032
   GGGGCTGCAG GGGTATTCCT GTATAAGACT TAGAGGTAGT ATGATATCTC AGATTTCTGC    2092
   CTTAAAGGGC TTTCTTTTTA GAAATAGTTT CTTTTATTGC CCTTAGAAGA TCACCCCCAG    2152
   GAAGAGTATG AGCTATCTTT TCTACATTTC TTTTCCTAGG AATATTCTTA TCCATTTCTT    2212
   ATATACATTT CTTTTGGGAG GGAGTTTTTA TGCTATAGTT GCTGGTATTT ATGTAAAGGG    2272
   ACCATTACTA AGTGTATTTC TCTAGCATAT TATGTTTAAG GGACGTGTGT A             2323
(2) INFORMATION FOR SEQ ID NO:4:
   (i) SEQUENCE CHARACTERISTICS:
      (A) LENGTH: 160 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Leu Val Leu Leu Ala Gly Leu Phe Val Val His Ile Ala Thr Ala
    1               5                  10                  15
   Ile Met Leu Phe Val Ser Thr Ile Ala Asn Val Trp Met Val Ala Asp
               20                  25                  30
   Tyr Ala Asn Ala Ser Val Gly Leu Trp Lys Asn Cys Thr Gly Gly Asn
           35                  40                  45
   Cys Asp Gly Ser Leu Ser Tyr Gly Asn Glu Asp Ala Ile Lys Ala Val
       50                  55                  60
   Gln Ala Phe Met Ile Leu Ser Ile Ile Phe Ser Ile Ile Ser Leu Val
    65                  70                  75                  80
   Val Phe Val Phe Gln Leu Phe Thr Met Glu Lys Gly Asn Arg Phe Phe
                    85                  90                  95
   Leu Ser Gly Ser Thr Met Leu Val Cys Trp Leu Cys Ile Leu Val Gly
               100                 105                 110
   Val Ser Ile Tyr Thr His His Tyr Ala His Ser Glu Gly Asn Phe Asn
           115                 120                 125
   Ser Ser Ser His Gln Gly Tyr Cys Phe Ile Leu Thr Trp Ile Cys Phe
       130                 135                 140
   Cys Phe Ser Phe Ile Ile Gly Ile Leu Tyr Met Val Leu Arg Lys Lys
   145                 150                 155                 160
(2) INFORMATION FOR SEQ ID NO:5:
   (i) SEQUENCE CHARACTERISTICS:
      (A) LENGTH: 1725 base pairs
      (B) TYPE: nucleic acid
      (C) STRANDEDNESS: single
      (D) TOPOLOGY: linear
   (ii) MOLECULE TYPE: CdNA
   (ix) FEATURE:
      (A) NAME/KEY: CDS
      (B) LOCATION: 8..478
   (xi) SEQUENCE DESCRIPTION: SEQ ID NO:5:
   AGCCAAC ATG TTG GTA TTG CTG GCT GGT ATC TTT GTG GTC CAC ATC GCT      49
           Met Leu Val Leu Leu Ala Gly Ile Phe Val Val His Ile Ala
            1               5                  10
   ACT GTT ATT ATG CTA TTT GTT AGC ACC ATT GCC AAT GTC TGG TTG GTT      97
   Thr Val Ile Met Leu Phe Val Ser Thr Ile Ala Asn Val Trp Leu Val
    15                  20                  25                  30
   TCC AAT ACG GTA GAT GCA TCA GTA GGT CTT TGG AAA AAC TGT ACC AAC 145
   Ser Asn Thr Val Asp Ala Ser Val Gly Leu Trp Lys Asn Cys Thr Asn
   35 40 45
   ATT AGC TGC AGT GAC AGC CTG TCA TAT GCC AGT GAA GAT GCC CTC AAG 193
   Ile Ser Cys Ser Asp Ser Leu Ser Tyr Ala Ser Glu Asp Ala Leu Lys
   50 55 60
   ACA GTG CAG GCC TTC ATG ATT CTC TCT ATC ATC TTC TGT GTC ATT GCC 241
   Thr Val Gln Ala Phe Met Ile Leu Ser Ile Ile Phe Cys Val Ile Ala
   65 70 75
   CTC CTG GTC TTC GTG TTC CAG CTC TTC ACC ATG GAG AAG GGA AAC CGG 289
   Leu Leu Val Phe Val Phe Gln Leu Phe Thr Met Glu Lys Gly Asn Arg
   80 85 90
   TTC TTC CTC TCA GGG GCC ACC ACA CTG GTG TGC TGG CTG TGC ATT CTT 337
   Phe Phe Leu Ser Gly Ala Thr Thr Leu Val Cys Trp Leu Cys Ile Leu
   95 100 105 110
   GTG GGG GTG TCC ATC TAC ACT AGT CAT TAT GCG AAT CGT GAT GGA ACG 385
   Val Gly Val Ser Ile Tyr Thr Ser His Tyr Ala Asn Arg Asp Gly Thr
   115 120 125
   CAG TAT CAC CAC GGC TAT TCC TAC ATC CTG GGC TGG ATC TGC TTC TGC 433
   Gln Tyr His His Gly Tyr Ser Tyr Ile Leu Gly Trp Ile Cys Phe Cys
   130 135 140
   TTC AGC TTC ATC ATC GGC GTT CTC TAT CTG GTC CTG AGA AAG AAA 478
   Phe Ser Phe Ile Ile Gly Val Leu Tyr Leu Val Leu Arg Lys Lys
   145 150 155
   TAAGGCCGGA CGAGTTCATG GGGATCTGGG GGGTGGGGAG GAGGAAGCCG TTGAATCTGG 538
   GAGGGAAGTG GAGGTTGCTG TACAGGAAAA ACCGAGATAG GGGAGGGGGG AGGGGGAAGC 598
   AAAGGGGGGA GGTCAAATCC CAAACCATTA CTGAGGGGAT TCTCTACTGC CAAGCCCCTG 658
   CCCTGGGGAG AAAGTAGTTG GCTAGTACTT TGATGCTCCC TTGATGGGGT CCAGAGAGCC 718
   TCCCTGCAGC CACCAGACTT GGCCTCCAGC TGTTCTTAGT GACACACACT GTCTGGGGCC 778
   CCATCAGCTG CCACAACACC AGCCCCACTT CTGGGTCATG CACTGAGGTC CACAGACCTA 838
   CTGCACTGAG TTAAAATAGC GGTACAAGTT CTGGCAAGAG CAGATACTGT CTTTGTGCTG 898
   AATACGCTAA GCCTGGAAGC CATCCTGCCC TTCTGACCCA AAGCAAAACA TCACATTCCA 958
   GTCTGAAGTG CCTACTGGGG GGCTTTGGCC TGTGAGCCAT TGTCCCTCTT TGGAACAGAT 1018
   ATTTAGCTCT GTGGAATTCA GTGACAAAAT GGGAGGAGGA AAGAGAGTTT GTAAGGTCAT 1078
   GCTGGTGGGT TAGCTAAACC AAGAAGGAGA CCTTTTCACA ATGGAAAACC TGGGGGATGG 1138
   TCAGAGCCCA GTCGAGACCT CACACACGGC TGTCCCTCAT GGAGACCTCA TGCCATGGTC 1198
   TTTGCTAGGC CTCTTGCTGA AAGCCAAGGC AGCTCTTCTG GAGTTTCTCT AAAGTCACTA 1258
   GTGAACAATT CGGTGGTAAA AGTACCACAC AAACTATGGG ATCCAAGGGG CAGTCTTGCA    1318
   ACAGTGCCAT GTTAGGGTTA TGTTTTTAGG ATTCCCCTCA ATGCAGTCAG TGTTTCTTTT    1378
   AAGTATACAA CAGGAGAGAG ATGGACATGG CTCATTGTAG CACAATCCTA TTACTCTTCC    1438
   TCTAACATTT TTGAGGAAGT TTTGTCTAAT TATCAATATT GAGGATCAGG GCTCCTAGGC    1498
   TCAGTGGTAG CTCTGGCTTA GACACCACCT GGAGTGATCA CCTCTTGGGG ACCCTGCCTA    1558
   TCCCACTTCA CAGGTGAGGC ACCGGAATTC TGGAAGCTGA TTAAAACACA CATAAACCAA    1618
   AACCAAACAA CAGGCCCTTG GGTGAAAGGT GCTATATAAT TGTGAAGTAT TAAGCCTACC    1678
   GTATTTCAGC CATGATAAGA ACAGAGTGCC TGCATTCCCA GGAAAAT                  1725
(2) INFORMATION FOR SEQ ID NO:6:
   (i) SEQUENCE CHARACTERISTICS:
      (A) LENGTH: 157 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Leu Val Leu Leu Ala Gly Ile Phe Val Val His Ile Ala Thr Val
    1               5                   10                  15
   Ile Met Leu Phe Val Ser Thr Ile Ala Asn Val Trp Leu Val Ser Asn
               20                  25                  30
   Thr Val Asp Ala Ser Val Gly Leu Trp Lys Asn Cys Thr Asn Ile Ser
           35                  40                  45
   Cys Ser Asp Ser Leu Ser Tyr Ala Ser Glu Asp Ala Leu Lys Thr Val
       50                  55                  60
   Gln Ala Phe Met Ile Leu Ser Ile Ile Phe Cys Val Ile Ala Leu Leu
   65                  70                  75                  80
   Val Phe Val Phe Gln Leu Phe Thr Met Glu Lys Gly Asn Arg Phe Phe
                   85                  90                  95
   Leu Ser Gly Ala Thr Thr Leu Val Cys Trp Leu Cys Ile Leu Val Gly
               100                 105                 110
   Val Ser Ile Tyr Thr Ser His Tyr Ala Asn Arg Asp Gly Thr Gln Tyr
           115                 120                 125
   His His Gly Tyr Ser Tyr Ile Leu Gly Trp Ile Cys Phe Cys Phe Ser
       130                 135                 140
   Phe Ile Ile Gly Val Leu Tyr Leu Val Leu Arg Lys Lys
   145                 150                 155
(2) INFORMATION FOR SEQ ID NO:7:
   (i) SEQUENCE CHARACTERISTICS:
      (A) LENGTH: 173 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:7:
   Met Tyr Ser Phe Met Gly Gly Gly Leu Phe Cys Ala Trp Val Gly Thr
   1               5                   10                  15
   Ile Leu Leu Val Val Ala Thr Ala Thr Asp His Trp Met Gln Tyr Arg
               20                  25                  30
   Leu Ser Gly Ser Phe Ala His Gln Gly Leu Trp Arg Tyr Cys Leu Gly
           35                  40                  45
   Asn Lys Cys Phe Leu Gln Thr Glu Ser Ile Ala Tyr Trp Asn Ala Thr
       50                  55                  60
   Arg Ala Phe Met Ile Leu Ser Ala Leu Cys Ala Thr Ser Gly Ile Ile
   65                  70                  75                  80
   Met Gly Val Leu Ala Phe Ala Gln Gln Ser Thr Phe Thr Arg Leu Ser
                   85                  90                  95
   Arg Pro Phe Ser Ala Gly Ile Met Phe Phe Ala Ser Thr Leu Phe Val
               100                 105                 110
   Leu Leu Ala Leu Ala Ile Tyr Thr Gly Val Thr Val Ser Phe Leu Gly
           115                 120                 125
   Arg Arg Phe Gly Asp Trp Arg Phe Ser Trp Ser Tyr Ile Leu Gly Trp
       130                 135                 140
   Val Ala Leu Leu Met Thr Phe Phe Ala Gly Ile Phe Tyr Met Cys Ala
   145                 150                 155                 160
   Tyr Arg Met His Glu Cys Arg Arg Leu Ser Thr Pro Arg
                   165                 170